# EUROPEAN PATENT APPLICATION

(11) **EP 4 516 213 A1**
(43) Date of publication of application: **05.03.2025**
(21) Application number: 23796408.5
(22) Date of filing: 25.04.2023
(51) Int. Cl.: A61B 5/021, A61B 5/02, A61B 5/11, A61B 5/16

(54) **BIOLOGICAL STATE EVALUATION DEVICE, BIOLOGICAL STATE EVALUATION METHOD, COMPUTER PROGRAM, AND RECORDING MEDIUM**

(30) Priority: 26.04.2022 JP 2022072790
(71) Applicant: Delta Tooling Co., Ltd., Hiroshima-shi, Hiroshima 736-0084 (JP)
(72) Inventor: FUJITA Etsunori, Hiroshima-shi, Hiroshima 736-0084 (JP); OGURA Yumi, Hiroshima-shi, Hiroshima 736-0084 (JP); MAEDA Shinichiro, Hiroshima-shi, Hiroshima 736-0084 (JP); NOBUHIRO Yoshika, Hiroshima-shi, Hiroshima 736-0084 (JP); UCHIKAWA Ryuichi, Hiroshima-shi, Hiroshima 736-0084 (JP); ONODA Ryo, Hiroshima-shi, Hiroshima 736-0084 (JP)
(74) Representative: Hoffmann Eitle
(86) International application number: PCT/JP2023/016368
(87) International publication number: WO 2023/210668

(57) **Abstract**

Accuracy of the inference of a biological state associated with a blood pressure is improved. Through biological signal data analysis, heart rate variability information is found and represented on log-log axes, a quartic function is set for a waveform resulting from analysis of the information, and one of two types of gradient values obtained based on different criteria, i.e., a gradient (GI) between points of inflection and a gradient (GT) of a tangent at a point of inflection (a GI value and a GT value), is employed as a final analysis result, depending on the number of extreme values in the quartic function. A cardiac output influenced by two factors, i.e., nervous system regulation which influences a heart rate and an arterial blood pressure which influences a stroke volume, undergoes increases/decreases due to the respective two factors. However, owing to the employment of one of the GI value and the GT value, an increase/decrease in the influence of each of these two factors is reflected.

## Description

### Technical Field

The present invention relates to a biological state evaluation device that evaluates the biological state of a person in relation to a blood pressure, a biological state evaluation method, a computer program, and a recording medium.

### Background Art

In Patent Document 1, the present applicant has disclosed a blood pressure estimation device that analyzes biological signal data obtained from a biological signal detection sensor including a three-dimensional knitted fabric, captures information on vibration generated in a living body by blood flow variability in a period from a ventricular filling period to an isovolumetric systole, finds an index indicating its fluctuation by using a Lorenz plot method, and estimates a blood pressure value from the index (Patent Document 1).

Specifically, this is a technique of capturing a biological signal on a dorsal body surface from the biological signal detection sensor, applying predetermined frequency-band filtering to its time waveform, to thereby obtain a filtered waveform in which a cardiac cycle is made apparent, and collating this filtered waveform with simultaneously measured electrocardiogram waveform data obtained from an electrocardiograph, identifying a waveform component in the period from the ventricular filling period to the isovolumetric systole, and thereafter, defining, as a reference, a slope of a group of many points that are plotted in a predetermined measurement time using the Lorenz plot method, newly configuring a time waveform regarding an angle difference between the reference slope and a slope of a point group in a measurement time shorter than the predetermined measurement time, frequency-analyzing the time waveform, representing the frequency analysis result on log-log axes, finding a regression line regarding a range from VLF to LF in the analyzed waveform, defining a slope of the regression line as a fractal slope, and estimating the blood pressure from the fractal slope.

### Prior Art Document

### Patent Document

Patent Document 1: Japanese Patent Application Laid-open No. 2019-122502

### Summary of the Invention

### Problems to Be Solved by the Invention

According to Patent Document 1, it is possible to estimate a blood pressure from the biological signals collected from the back of a person without restraining the person. Accordingly, it is not necessary to wind a cuff around the upper part of an arm, leading to easy measurement, and another merit is that the measurement without restraining enables the successive measurement of the blood pressure.

In the technique of Patent Document 1, however, erroneous detection may occur, for example, a subject who should be hypertensive is determined as normotensive or vice versa, which has given rise to a demand for an improvement in detection accuracy.

In considering the accuracy improvement, the present inventor paid attention to the fact that a cardiac output is determined by a heart rate and a stroke volume.

A heart rate is regulated by sympathetic nerves and parasympathetic nerves distributed in the sinus node and the atrioventricular node. On the other hand, a stroke volume is determined by preload, afterload, contractility, and so on. A cardiac output influenced by two factors, which are nervous system regulation having an influence on a heart rate and an arterial pressure having an influence on a stroke volume, undergoes increases/decreases due to the respective two factors, and the increases/decreases become points of inflection. Therefore, a cardiac output has two points of inflection due to the two factors, and a cardiac output having two points of inflection is represented by a quartic function and extreme values.

It is known that heart rate variability at rest has chaotic characteristics and has 1/fⁿ fluctuation. If the quartic function is set in a frequency band where the 1/fⁿ fluctuation is exhibited, a combination of an increase/decrease between two points of inflection and a concave-convex function determines a gradient between points of inflection (Gradient of inflection: GI) and a gradient of a tangent at a point of inflection (Gradient of tangent: GT). Then, the GI value or the GT value is determined by a value of n of 1/fⁿ, and blood pressure is also determined by the value of n.

A GI value close to 1/f is considered to correlate with an arterial pressure to which the principle of least energy action is applied, and a GT value that is an outlier with respect to 1/f is considered to correlate with an arterial pressure indicating hypertension or hypotension. The GI value and the GT value appear in the VLF band and the LF band in which thermoregulation and nervous system regulation are reflected.

The present invention was made in consideration of the above and has an object to improve blood pressure inference accuracy by making the influences of the two factors determining a cardiac output reflected in the determination.

### Means for Solving the Problems

To solve the aforesaid problem, the present invention provides a biological state evaluation device including:
an analyzing unit that analyzes biological signal data as sound/vibration information that is propagated from a surface of a body of a person through a three-dimensional knitted fabric of a biological signal detection sensor having the three-dimensional knitted fabric and a microphone, to be detected by the microphone while the biological signal detection sensor is in contact with the body; and
an evaluating unit that accesses a database in which correlation data of analysis results found by the analyzing unit and blood pressure values found with a blood pressure monitor is constructed in advance, and collates an analysis result found by the analyzing unit regarding the biological signal data of an evaluation-target person with the correlation data to evaluate a biological state of the person in relation to a blood pressure,
wherein the analyzing unit analyzes the biological signal data, finds an analyzed waveform regarding heart rate variability information to represent the analyzed waveform on log-log axes, sets a quartic function for the analyzed waveform, finds a gradient between two points of inflection or a gradient of a tangent at a point of inflection depending on the number of extreme values in the quartic function, and outputs one of the gradients as the analysis result.

Preferably, based on whether the analysis result of the biological signal data of the evaluation-target person corresponds to the gradient between the two points of inflection or to the gradient of the tangent at the point of inflection, the evaluating unit determines blood pressure classification including at least distinction between normotension and hypertension.

Preferably, the evaluating unit collates a value of the gradient between the two points of inflection or the gradient of the tangent at the point of inflection that is the analysis result of the biological signal data of the evaluation-target person, with the correlation data, and infers a blood pressure value.

Preferably, the evaluating unit infers whether or not an autonomic nervous system is in disorder, based on whether or not analysis results of a plurality of biological signal data measured from the evaluation-target person at different times present a change conforming to a distribution trend of the correlation data.

Preferably, whether or not the autonomic nervous system is in disorder is inferred, using the plurality of biological signal data measured before and after an exercise load.

Preferably, in a case where the number of the extreme values in the quartic function is three and a concave-convex function is present sandwiching two points of inflection, the analyzing unit employs a gradient between the two points of inflection as the analysis result, and in a case where the number of the extreme values is two or less, the analyzing unit employs a gradient of a tangent at one of points of inflection as the analysis result.

Preferably, in a case where the number of the extreme values is two, the analyzing unit decides which one of tangents at points of inflection is to be employed in consideration of whether the point of inflection in a low-frequency band is higher or lower than a maximum value and whether or not both positive and negative gradients of tangents are present, and
in a case where the number of the extreme values is one and a concave-convex function is present sandwiching a point of inflection, the analyzing unit employs a tangent at a point of inflection that is not the extreme value.

Preferably, in a case where the analyzing unit finds through the analysis that the number of the extreme values in the quartic function is two and there are two gradients of tangents,
the evaluating unit
evaluates the biological state as having a possibility of an overload state in a case where it is confirmed that heart rates or blood pressures obtained at a predetermined time interval have a predetermined difference or more, and
evaluates the biological state as having a possibility of a physical condition sudden change in a case where it is confirmed that the heart rates and the blood pressures obtained at the predetermined time interval both have the predetermined difference or more.

Preferably, the analyzing unit finds, as the heart rate variability information, two peak-to-peak amplitudes from extreme values included in time phases of an atrial systole and a ventricular systole, finds a reference slope from a Lorenz plot in a reference time range by employing a Lorenz plot method, successively finds, in predetermined time windows with a predetermined overlap ratio, a relative slope which is a difference between a slope of each Lorenz plot created every predetermined time shorter than the reference time range and the reference slope, applies predetermined-frequency filtering to a time waveform of the obtained relative slope, frequency-analyzes a time waveform resulting from the filtering, converts a result of the frequency analysis to the analyzed waveform represented on the log-log axes, extracts a frequency band where a slope of a regression line of the analyzed waveform is close to 1/f, and finds the quartic function for a point group of the analyzed waveform in a range of the extracted frequency band.

Further, the present invention provides a biological state evaluation method of causing a computer to analyze biological signal data as sound/vibration information that is propagated from a surface of a body of a person through a three-dimensional knitted fabric of a biological signal detection sensor having the three-dimensional knitted fabric and a microphone, to be detected by the microphone while the biological signal detection sensor is in contact with the body, to evaluate a biological state of the person, the method including:
a procedure for analyzing the biological signal data, finding an analyzed waveform regarding heart rate variability information to represent the analyzed waveform on log-log axes, setting a quartic function for the analyzed waveform, finding a gradient between two points of inflection or a gradient of a tangent at a point of inflection depending on the number of extreme values in the quartic function, and outputting one of the gradients as an analysis result; and
a procedure for referring to correlation data, which is stored in a database in advance, of analysis results found through the execution of the analyzing procedure and blood pressure values found with a blood pressure monitor, and collating an analysis result of the biological signal data of an evaluation-target person with the correlation data to evaluate a biological state of the evaluation-target person in relation to a blood pressure.

Preferably, in the procedure for evaluating the biological state,
based on whether the analysis result of the biological signal data of the evaluation-target person corresponds to the gradient between the two points of inflection or to the gradient of the tangent at the point of inflection, blood pressure classification including at least distinction between normotension and hypertension is determined.

Preferably, in the procedure for evaluating the biological state,
a value of the gradient between the two points of inflection or the gradient of the tangent at the point of inflection that is the analysis result of the biological signal data of the evaluation-target person is collated with the correlation data, and a blood pressure value is inferred.

Preferably, in the procedure for evaluating the biological state,
whether or not an autonomic nervous system is in disorder is inferred based on whether or not analysis results of a plurality of biological signal data measured from the evaluation-target person at different times present a change conforming to a distribution trend of the correlation data.

Preferably, whether or not the autonomic nervous system is in disorder is inferred, using the plurality of biological signal data measured before and after an exercise load.

Preferably, in a case where it is found through the analysis that the number of the extreme values in the quartic function is two and there are two gradients of tangents,
in the procedure for evaluating the biological state,
the biological state is evaluated as having a possibility of an overload state in a case where it is confirmed that heart rates or blood pressures obtained at a predetermined time interval have a predetermined difference or more, and
the biological state is evaluated as having a possibility of a physical condition sudden change in a case where it is confirmed that the heart rates and the blood pressures obtained at the predetermined time interval both have the predetermined difference or more.

Further, the present invention provides a computer program causing a computer to analyze biological signal data as sound/vibration information that is propagated from a surface of a body of a person through a three-dimensional knitted fabric of a biological signal detection sensor having the three-dimensional knitted fabric and a microphone, to be detected by the microphone while the biological signal detection sensor is in contact with the body, to evaluate a biological state of the person, the program causing the computer to execute:
a procedure for analyzing the biological signal data, finding an analyzed waveform regarding heart rate variability information to represent the analyzed waveform on log-log axes, setting a quartic function for the analyzed waveform, finding a gradient between two points of inflection or a gradient of a tangent at a point of inflection depending on the number of extreme values in the quartic function, and outputting one of the gradients as an analysis result; and
a procedure for referring to correlation data, which is stored in a database in advance, of analysis results found through the execution of the analyzing procedure and blood pressure values found with a blood pressure monitor, and collating an analysis result of the biological signal data of an evaluation-target person with the correlation data to evaluate a biological state of the evaluation-target person in relation to a blood pressure,
thereby causing the computer to function as a biological state evaluation device.

Preferably, in the procedure for evaluating the biological state,
based on whether the analysis result of the biological signal data of the evaluation-target person corresponds to the gradient between the two points of inflection or to the gradient of the tangent at the point of inflection, blood pressure classification including at least distinction between normotension and hypertension is determined.

Preferably, in the procedure for evaluating the biological state,
a value of the gradient between the two points of inflection or the gradient of the tangent at the point of inflection that is the analysis result of the biological signal data of the evaluation-target person is collated with the correlation data, and a blood pressure value is inferred.

Preferably, in the procedure for evaluating the biological state,
whether or not an autonomic nervous system is in disorder is inferred based on whether or not analysis results of a plurality of biological signal data measured from the evaluation-target person at different times present a change conforming to a distribution trend of the correlation data.

Preferably, whether or not the autonomic nervous system is in disorder is inferred, using the plurality of biological signal data measured before and after an exercise load.

Preferably, in the procedure for outputting the analysis result,
in a case where the number of the extreme values in the quartic function is three and a concave-convex function is present sandwiching two points of inflection, a gradient between the two points of inflection is employed as the analysis result, and in a case where the number of the extreme values is two or less, a gradient of a tangent at one of points of inflection is employed as the analysis result.

Preferably, in the procedure for outputting the analysis result,
in a case where the number of the extreme values is two, it is decided which one of tangents at points of inflection is to be employed in consideration of whether the point of inflection in a low-frequency band is higher or lower than a maximum value and whether or not both positive and negative gradients of tangents are present, and
in a case where the number of the extreme values is one and a concave-convex function is present sandwiching a point of inflection, a tangent at a point of inflection that is not the extreme value is employed.

Preferably, in a case where it is found through the analysis that the number of the extreme values in the quartic function is two and there are two gradients of tangents,
in the procedure for evaluating the biological state,
the biological state is evaluated as having a possibility of an overload state in a case where it is confirmed that heart rates or blood pressures obtained at a predetermined time interval have a predetermined difference or more, and
the biological state is evaluated as having a possibility of a physical condition sudden change in a case where it is confirmed that the heart rates and the blood pressures obtained at the predetermined time interval both have the predetermined difference or more.

Preferably, in the procedure for outputting the analysis result,
as the heart rate variability information, two peak-to-peak amplitudes are found from extreme values included in time phases of an atrial systole and a ventricular systole, a reference slope is found from a Lorenz plot in a reference time range by employing a Lorenz plot method, a relative slope which is a difference between a slope of each Lorenz plot created every predetermined time shorter than the reference time range and the reference slope is successively found in predetermined time windows with a predetermined overlap ratio, predetermined-frequency filtering is applied to a time waveform of the obtained relative slope, a time waveform resulting from the filtering is frequency-analyzed, a result of the frequency analysis is converted to the analyzed waveform represented on the log-log axes, a frequency band where a slope of a regression line of the analyzed waveform is close to 1/f is extracted, and the quartic function is found for a point group of the analyzed waveform in a range of the extracted frequency band.

Further, the present invention provides a computer-readable recording medium in which the aforesaid computer program is recorded.

### Effect of the Invention

According to the present invention, the biological signal data is analyzed, the heart rate variability information is found and represented on the log-log axes, the quartic function is set for the analyzed waveform, and one of the two kinds of gradient values (the GI value and the GT value) obtained based on different criteria, i.e., the gradient (GI) of inflection and the gradient (GT) of the tangent at the point of inflection, is employed as the final analysis result, depending on the number of extreme values in the quartic function. As described above, a cardiac output influenced by two factors, i.e., the regulation of the nervous system which influences a heart rate and an arterial blood pressure which influences a stroke volume, undergoes increases/decreases due to the respective two factors. However, owing to the employment of one of the GI value and the GT value, an increase/decrease of the influence of each of these two factors is reflected. As a result, based on whether the GI value is employed or the GT value is employed, whether the blood pressure belongs to a normotension region or belongs to a region of hypertension or the like other than normotension is clearly distinguished, making it possible to improve the blood pressure inference accuracy as compared with the aforesaid conventional art.

Further, in the present invention, the three-dimensional knitted fabric is used as the biological signal detection sensor, which has recyclability, and its surface is covered with a low-cost synthetic resin film. Accordingly, the film which comes into contact with a person is disposable. Therefore, it is suitable for use in hospitals, public transportation facilities, and so on requiring a strong measure against infectious diseases.

### Brief Description of Drawings

[FIGs. 1] FIG. 1(a) is an external perspective view of a biological signal detection sensor (4SR) used in an embodiment of the present invention, FIG. 1(b) is its external perspective view with an air pack and a gel pack separated, and FIG. 1(c) is its sectional view.
[FIG. 2] FIG. 2 is a view schematically illustrating an attachment position of the biological signal detection sensor when an acoustic pulse wave from the posterior chest surface (R-APW) is collected.
[FIG 3] FIG. 3 is a block diagram illustrating a schematic configuration of a biological state evaluation device according to the embodiment of the present invention.
[FIG. 4] FIG. 4 is a block diagram illustrating a schematic configuration of an analyzing unit of the biological state evaluation device illustrated in FIG. 3.
[FIG. 5] FIG. 5 is an explanatory flowchart of an analyzing step in the analyzing unit.
[FIG. 6] FIG. 6 is a chart illustrating data output in steps in order along the flowchart in FIG. 5.
[FIGs. 7] FIGs. 7(a), (b) are explanatory charts of how a regression curve which is a graph represented by a quartic function is found.
[FIG. 8] FIG. 8 is a chart illustrating correlation data of a systolic blood pressure (SBP) and a GI value or a GT value (SBP-GIGT correlation chart) in Experiment 1.
[FIGs. 9] FIGs. 9(a) to (d) are charts illustrating frequency analysis results and regression curves represented by quartic function graphs, for explaining cases where a GI value and GT values are derived using examples of data of subjects in group A in Experiment 1.
[FIGs. 10] FIGs. 10(a) to (d) are charts illustrating time waveforms of ECGs (electrocardiograms), PCGs (phonocardiograms), PPGs (finger plethysmograms), and RCs corresponding to FIGs. 9(a) to (d) respectively.
[FIGs. 11] FIGs. 11(a) to (d) are charts illustrating frequency analysis results and regression curves represented by quartic function graphs, for explaining cases where a GI value and GT values are derived using an example of data of subjects in group B in Experiment 1.
[FIGs. 12] FIGs. 12(a) to (d) are charts illustrating time waveforms of ECGs (electrocardiograms), PCGs (phonocardiograms), PPGs (finger plethysmograms), and RCs corresponding to FIGs. 11(a) to (d) respectively.
[FIG. 13] FIG. 13 is a chart in which an independent variable n on the horizontal axis which is a differential coefficient (a gradient on log-log axes: a gradient between points of inflection (GI value), a gradient of a tangent at a point of inflection (GT value)) of a quartic function graph and an inverse trigonometric function of n on the vertical axis are represented as Degree (Phase).
[FIG. 14] FIG. 14 illustrates an example of a chart where dense data is made sparse for easier view of its trend.

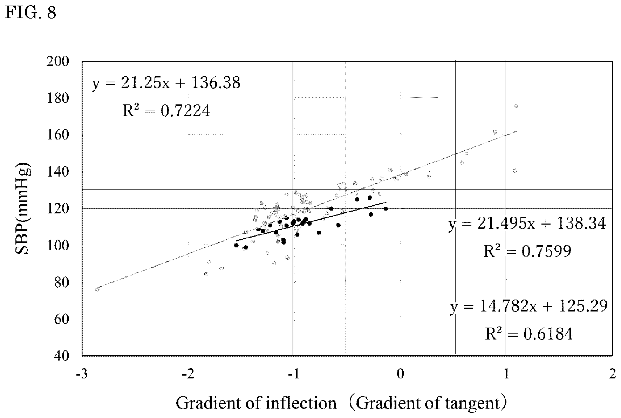

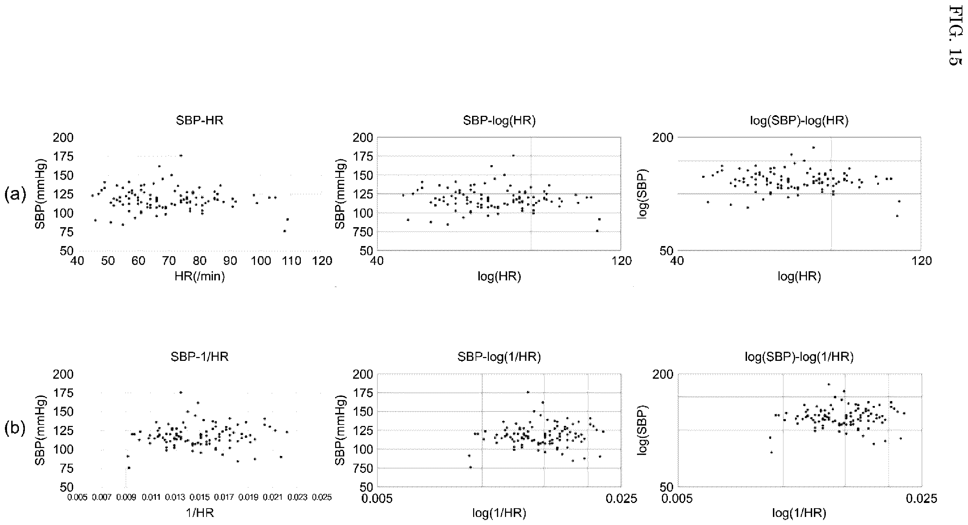

[FIGs. 15] FIG. 15(a) illustrates, in order from the left, a correlation chart of a systolic blood pressure and a heart rate (SBP-HR correlation chart) for which data used for the creation of FIG. 8 are used, a chart where the heart rate in the correlation chart is on a logarithmic scale, and a chart where both the systolic blood pressure and the heart rate in the correlation chart are on logarithmic scales. FIG. 15(b) illustrates, in order from the left, a correlation chart of the systolic blood pressure and a reciprocal of the heart rate (SBP-1/HR correlation chart) for which the data used for the creation of FIG. 8 are used, a chart where the heart rate in the correlation chart is on a logarithmic scale, and a chart where both the systolic blood pressure and the heart rate in the correlation chart are on logarithmic scales.
[FIG. 16] FIG. 16 is a chart illustrating an SBP-GIGT correlation chart where the measurement result of one subject is superimposed on a data group of many unspecified subjects.
[FIG. 17] FIG. 17 illustrates a correlation chart of a systolic blood pressure and a heart rate (SBP-HR correlation chart).
[FIGs. 18] FIG. 18(a) is an SBP-GIGT correlation chart regarding a case group where a slope is small with respect to a regression line of the SBP-GIGT correlation chart when blood pressure variability occurs, and FIG. 18(b) is an SBP-HR correlation chart.
[FIGs. 19] FIG. 19(a) is an SBP-GIGT correlation chart regarding a case group where an experimental value moves on a regression line of the SBP-GIGT correlation chart when blood pressure variability occurs, and FIG. 19(b) is an SBP-HR correlation chart.
[FIGs. 20] FIGs. 20(a) to (f) are an SBP-GIGT correlation chart, an SBP-HR correlation chart, and charts illustrating frequency analysis results, quartic function graphs, and GI values or GT values corresponding to respective postures, regarding two cases of the same subject (subject in his/her sixties) where his/her systolic blood pressure changes at the time of postural change.
[FIGs. 21] FIGs. 21(a) to (d) are an SBP-GIGT correlation chart, an SBP-HR correlation chart, and charts illustrating frequency analysis results, quartic function graphs, and GI values or GT values, regarding the same subject (subject in his/her sixties), when his/her systolic blood pressure suddenly changes.
[FIGs. 22] FIGs. 22(a) to (d) are charts illustrating GIGT values of the same subject when he/she took different postures, and time waveforms of ECGs, PCGs, PPGs, and RCs corresponding to the postures.
[FIGs. 23]

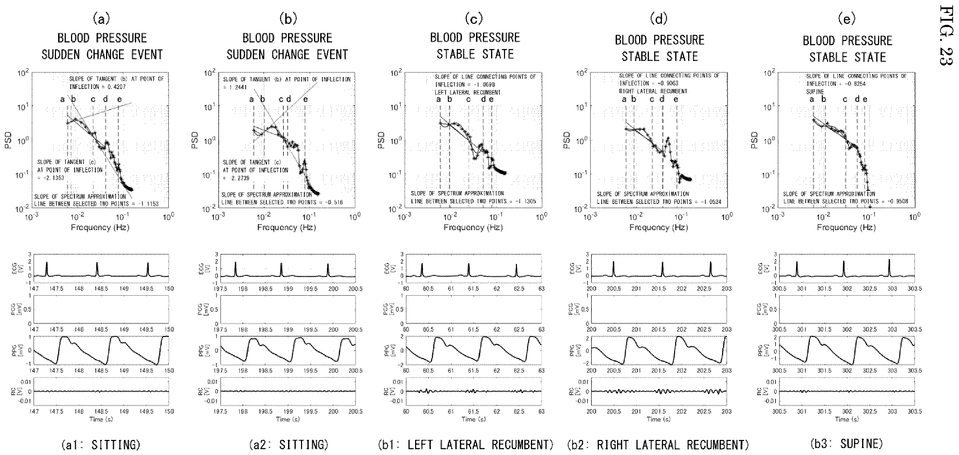

FIGs. 23(a) to (e) are charts illustrating GIGT values of the same subject when his/her blood pressure suddenly changes and when it is stable, and time waveforms of ECGs, PCGs, PPGs, and RCs corresponding to them.

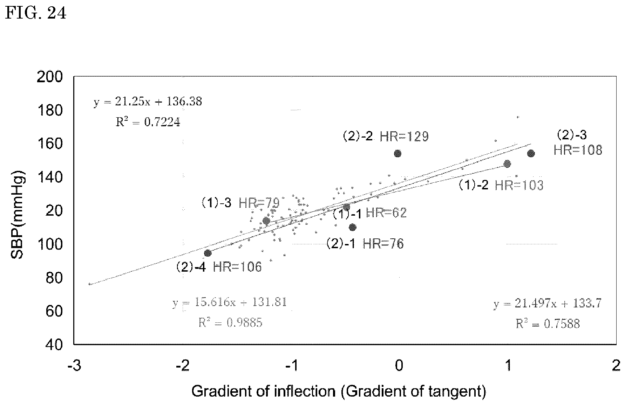

[FIG. 24] FIG. 24 is an SBP-GIGT correlation chart in an exercise stress test.

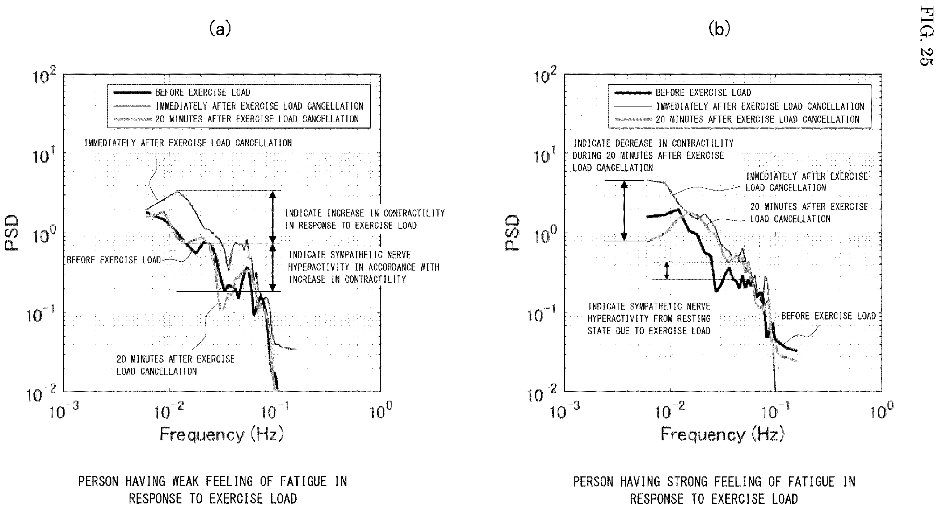

[FIGs. 25] FIG. 25(a) is a chart illustrating frequency analysis results of a subject in his/her twenties having a weak feeling of fatigue in response to an exercise load, and FIG. 25(b) is a chart illustrating frequency analysis results of a subject in his/her forties having a strong feeling of fatigue in response to an exercise load.

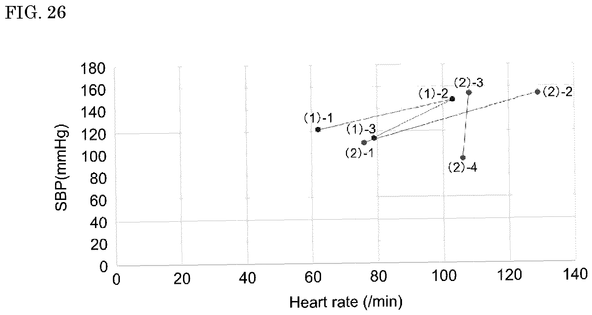

[FIG. 26] FIG. 26 is an SBP-HR correlation chart in the exercise stress test.

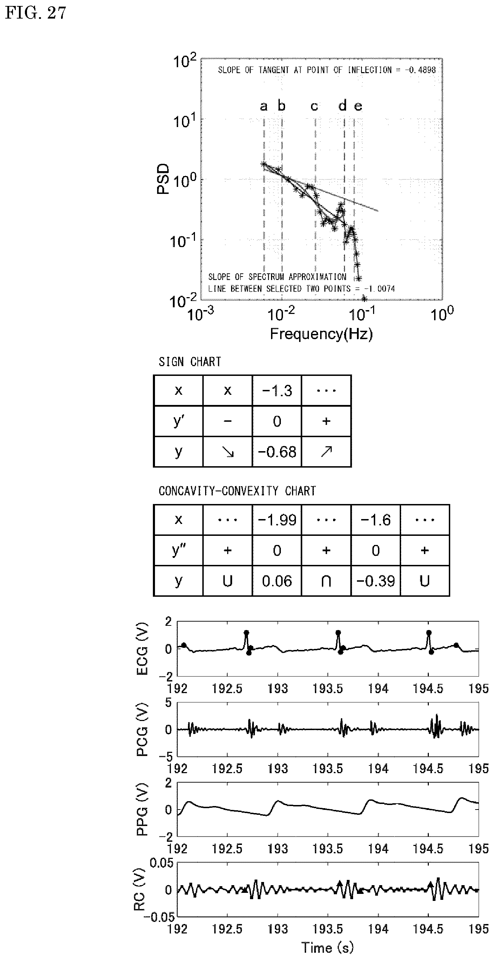

[FIG. 27] FIG. 27 is a chart illustrating a GIGT value, a sign chart, a concavity-convexity chart, and time waveforms of ECG, PCG, PPG, and RC, regarding the subject having a weak feeling of fatigue in response to the exercise load, at rest before the exercise load is given.

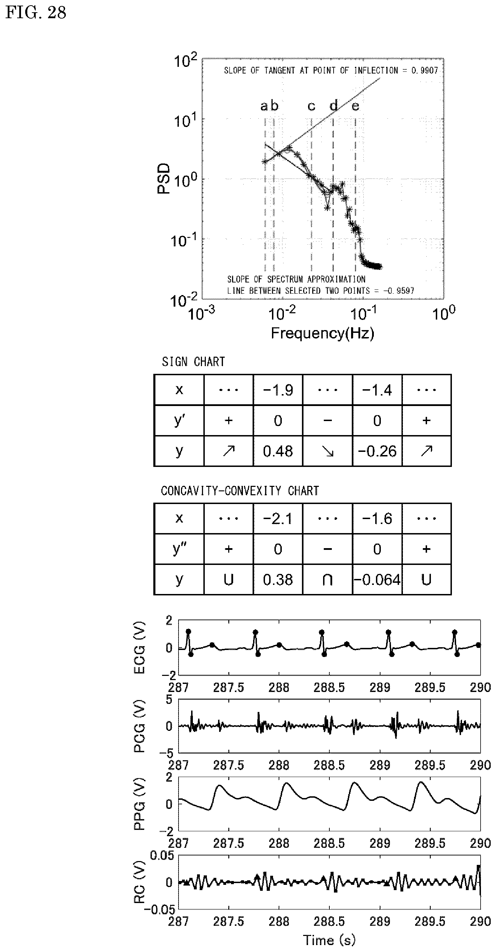

[FIG. 28] FIG. 28 is a chart illustrating a GIGT value, a sign chart, a concavity-convexity chart, and time waveforms of ECG, PCG, PPG, and RC, regarding the subject having a weak feeling of fatigue in response to the exercise load, at rest immediately after the cancellation of the exercise load.

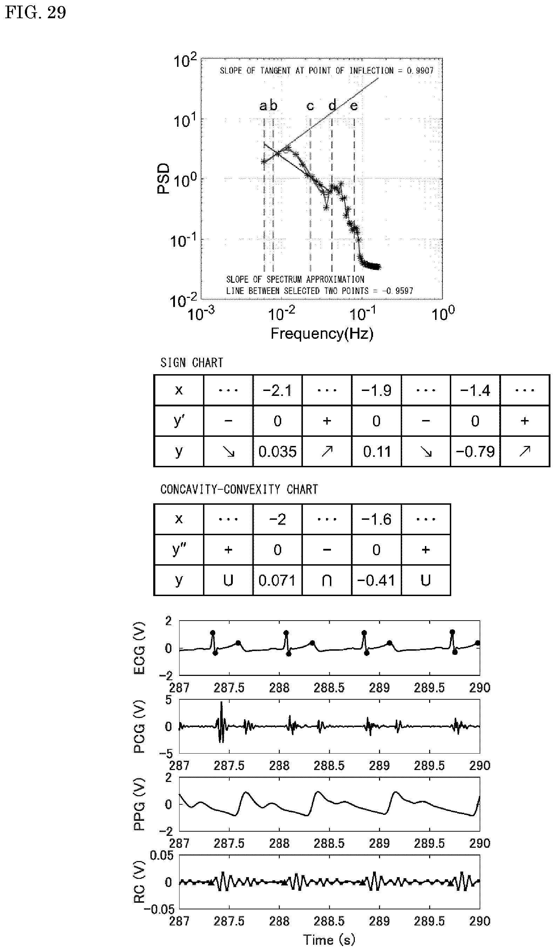

[FIG. 29] FIG. 29 is a chart illustrating a GIGT value, a sign chart, a concavity-convexity chart, and time waveforms of ECG, PCG, PPG, and RC, regarding the subject having a weak feeling of fatigue in response to the exercise load, at rest after twenty minutes passes from the cancellation of the exercise load.

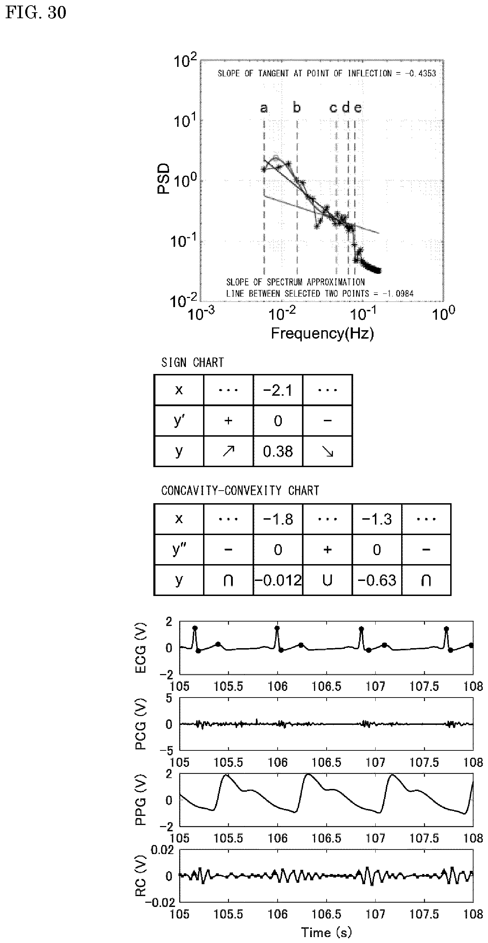

[FIG. 30] FIG. 30 is a chart illustrating a GIGT value, a sign chart, a concavity-convexity chart, and time waveforms of ECG, PCG, PPG, and RC, regarding the subject having a strong feeling of fatigue in response to the exercise load, at rest before the exercise load is given

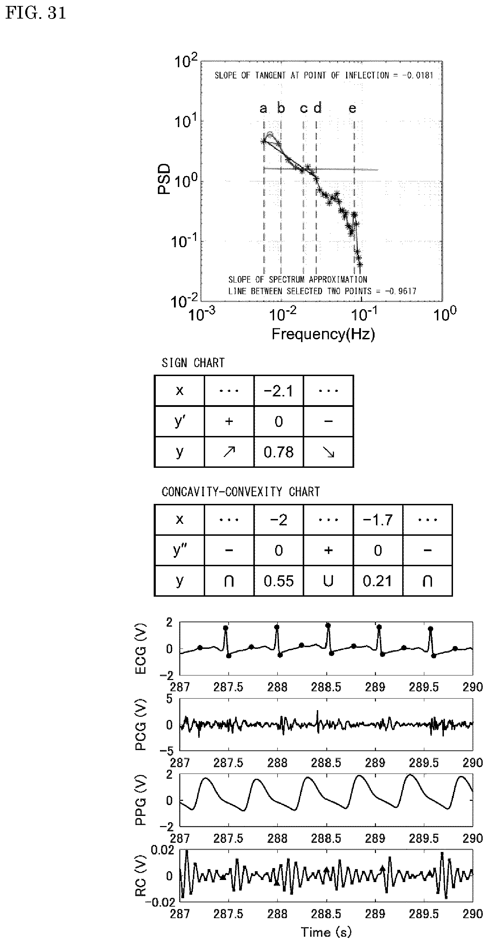

[FIG. 31] FIG. 31 is a chart illustrating a GIGT value, a sign chart, a concavity-convexity chart, and time waveforms of ECG, PCG, PPG, and RC, regarding the subject having a strong feeling of fatigue in response to the exercise load, at rest immediately after the cancellation of the exercise load.

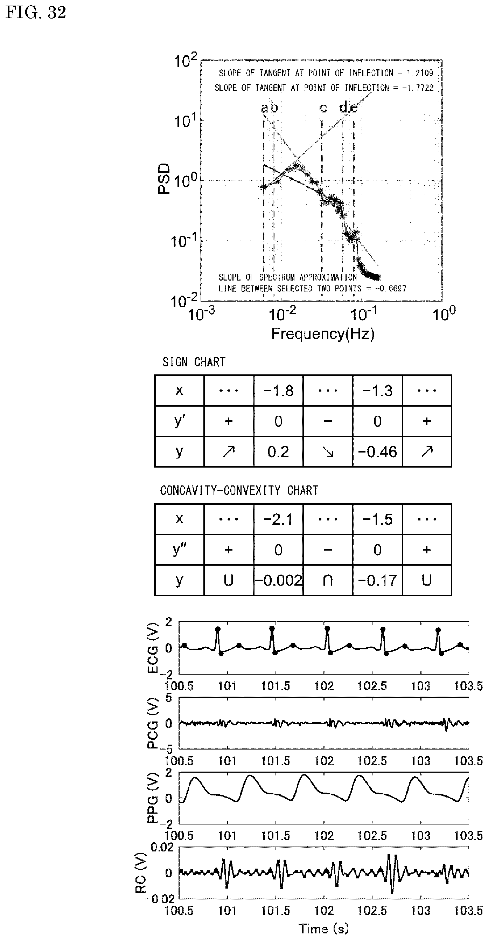

[FIG. 32] FIG. 32 is a chart illustrating a GIGT value, a sign chart, a concavity-convexity chart, and time waveforms of ECG, PCG, PPG, and RC, regarding the subject having a strong feeling of fatigue in response to the exercise load, at rest after twenty minutes passes from the cancellation of the exercise load

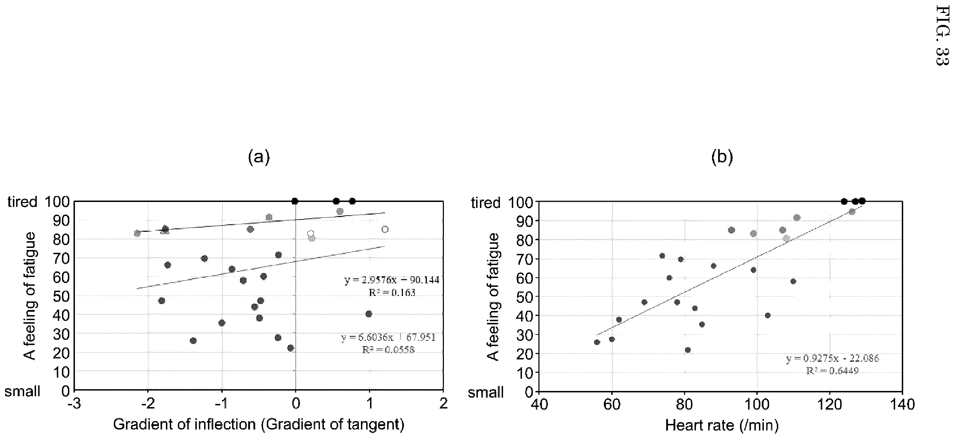

[FIGs. 33] FIG. 33(a) is a correlation chart of an index of the feeling of fatigue and a GIGT value, and FIG. 33(b) is a correlation chart of the index of the feeling of fatigue and a heart rate.

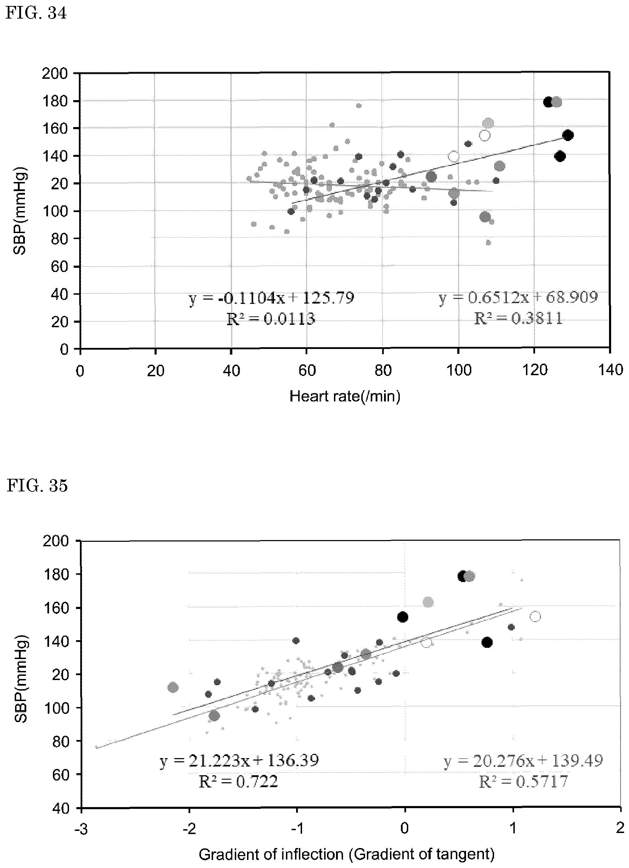

[FIG. 34] FIG. 34 is an SBP-HR correlation chart where distribution states in the resting state and after the exercise load are compared.
[FIG. 35] FIG. 35 is an SBP-GIGT correlation chart where distribution states in the resting state and in the exercise stress test are compared.

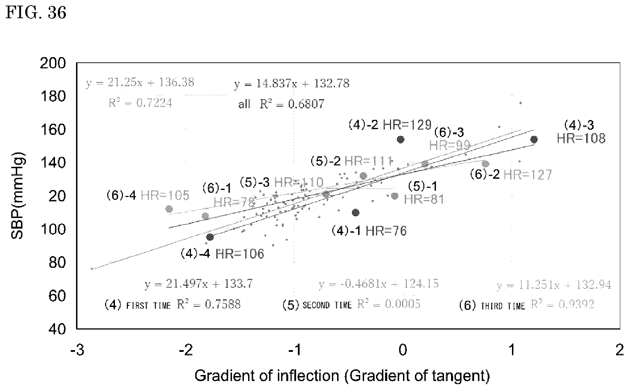

[FIG. 36] FIG. 36 is an SBP-GIGT correlation chart of the same person having a strong feeling of fatigue, where the results of three measurements conducted on different dates are illustrated in the same chart.

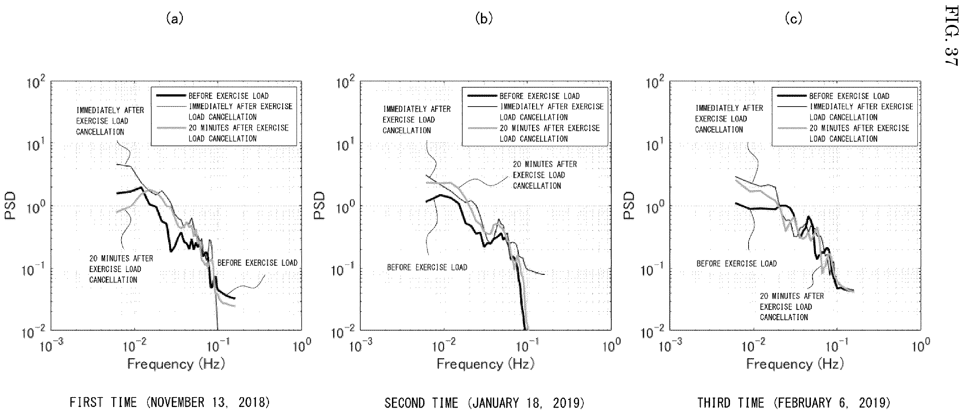

[FIGs. 37] FIGs. 37(a) to (c) are log-log plots of frequency-analyzed waveforms corresponding to the results of the three measurements in FIG. 36.

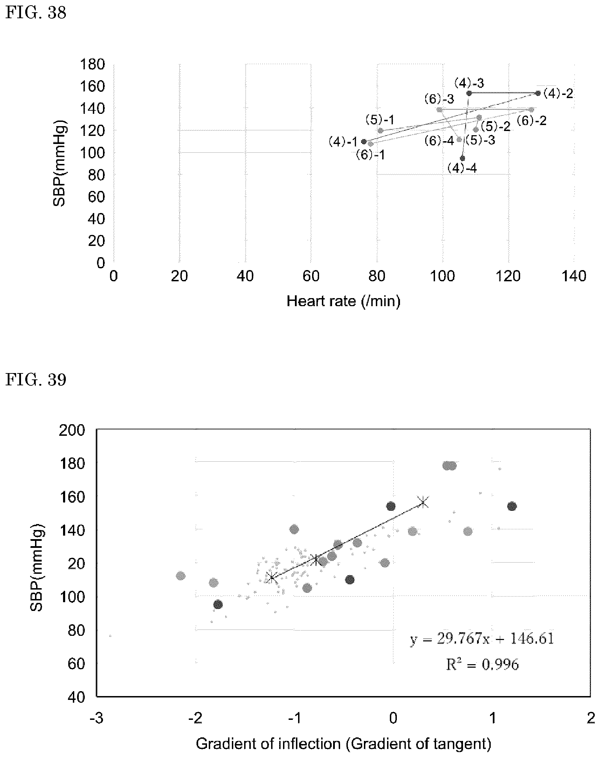

[FIG. 38] FIG. 38 is an SBP-HR correlation chart in the three measurements in FIG. 36.
[FIG. 39] FIG. 39 is an SBP-GIGT correlation chart where a regression line of a trajectory of a variation found from an average value of three subjects in a group with a strong feeling of fatigue is illustrated.

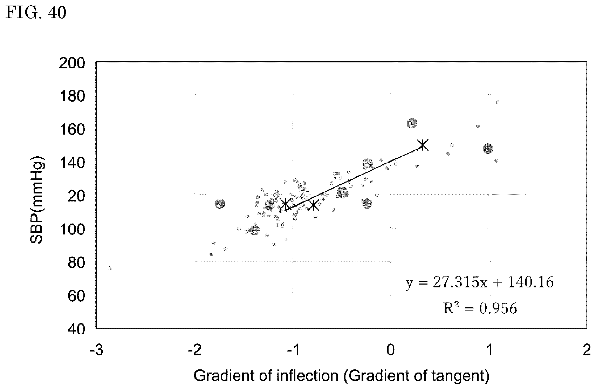

[FIG. 40] FIG. 40 is an SBP-GIGT correlation chart where a regression line of a trajectory of a variation found from an average value of three subjects in a group with a weak feeling of fatigue is illustrated.

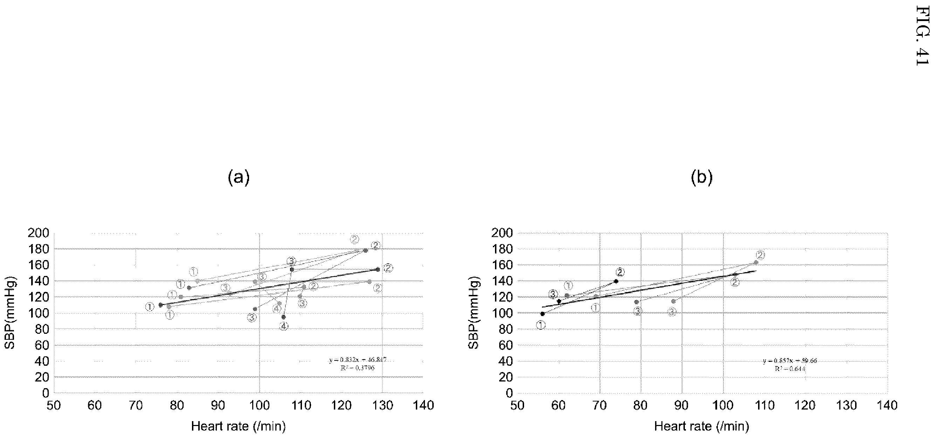

[FIGs. 41] FIG. 41(a) is a chart depicting trajectories of variations in the three subjects having a strong feeling of fatigue illustrated in FIG. 39, and FIG. 41(b) is a chart depicting variations in the three subjects having a weak feeling of fatigue illustrated in FIG. 40.

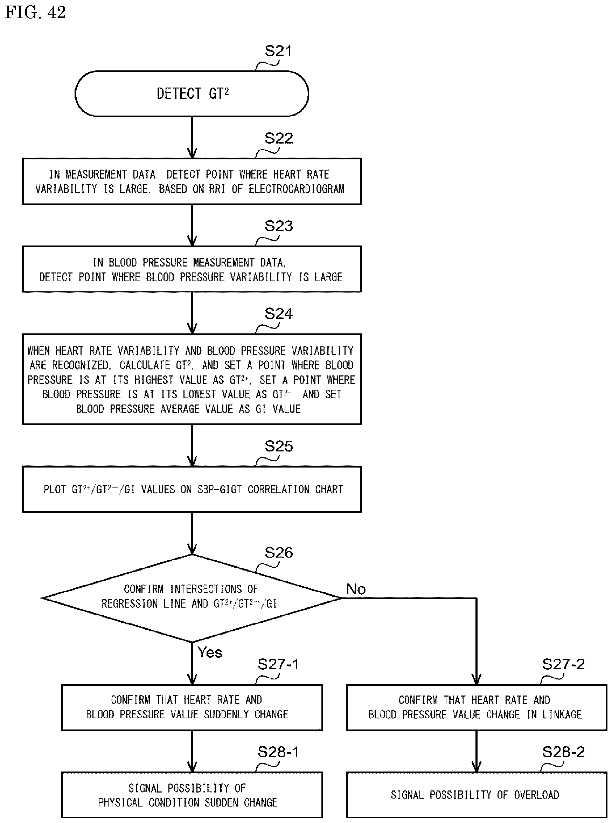

[FIG. 42] FIG. 42 is a flowchart illustrating a method of searching for a blood pressure sudden change case using GT².

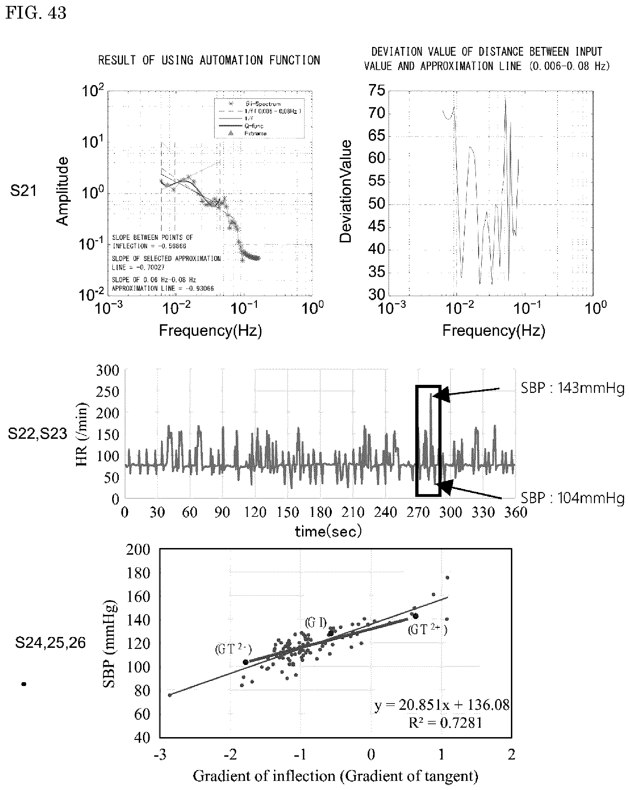

[FIG. 43] FIG. 43 is a chart illustrating graphs corresponding to steps S21 to S26 in FIG. 42.

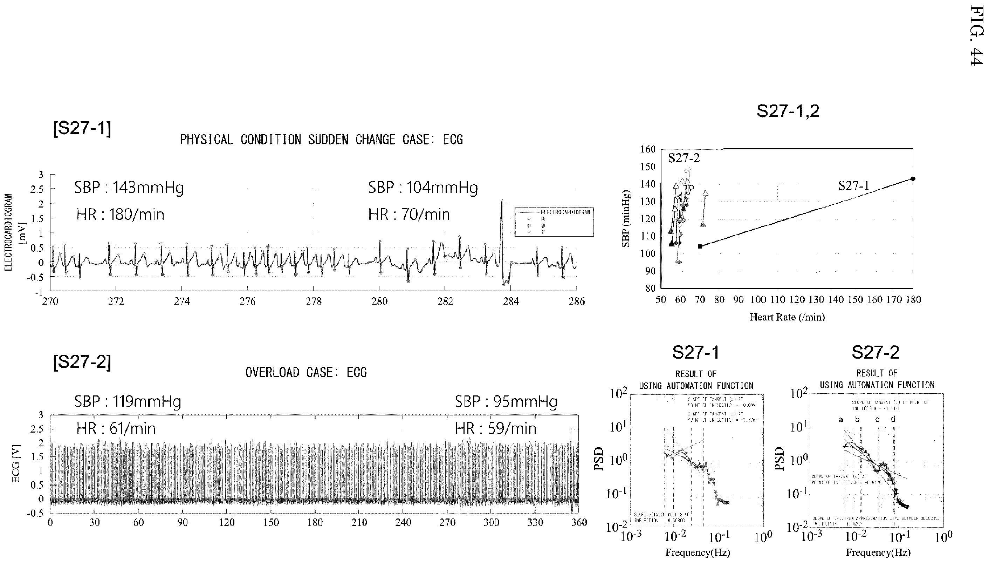

[FIG. 44] FIG. 44 is a chart illustrating graphs corresponding to steps S27-1, 2 in FIG. 42.

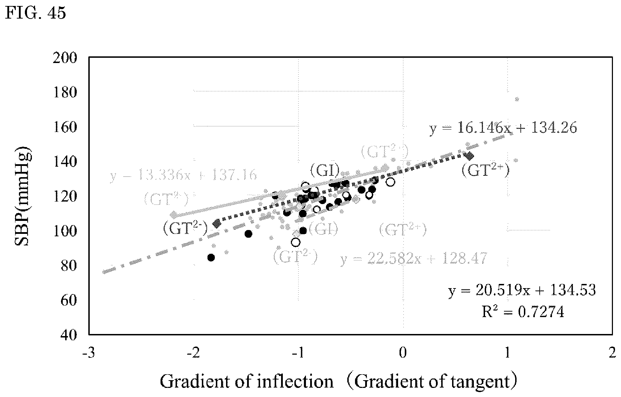

[FIG. 45] FIG. 45 illustrates an example of data of a subject with a blood pressure sudden change, and is an example of a chart where GT²⁺/GT²⁻/GI values are plotted on an SBP-GIGT correlation chart, to search for the subject with the blood pressure sudden change.

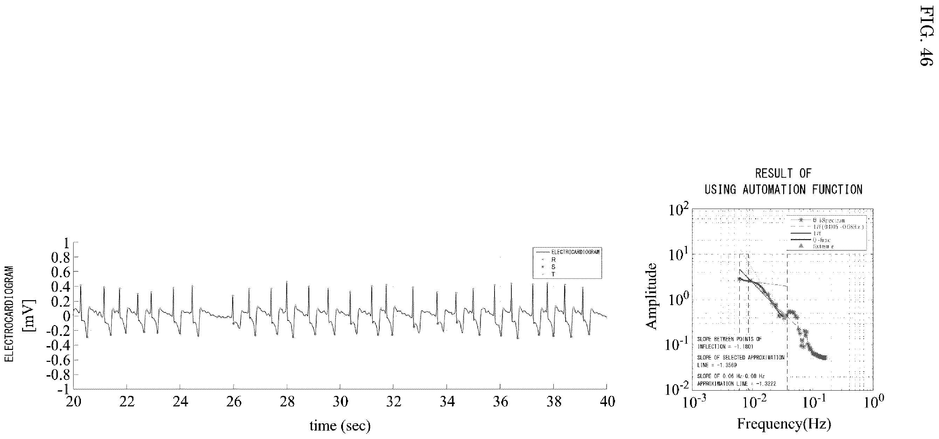

[FIG. 46] FIG. 46 is data of a case represented by the equation y = 13.336x +137.16 in FIG. 45.

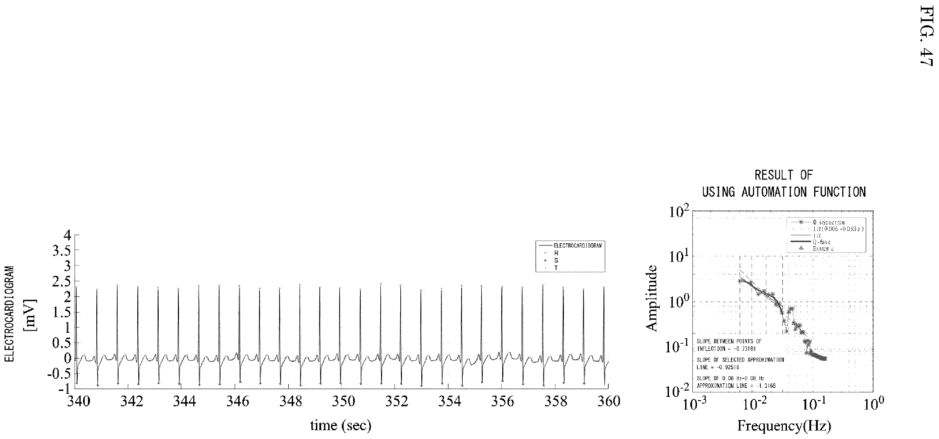

[FIG. 47] FIG. 47 is data of a case represented by the equation y = 22.582x +128.47 in FIG. 45.

### Modes for Carrying out the Invention

The present invention will be hereinafter described in more detail based on an embodiment of the present invention illustrated in the drawings.

### - Biological signal detection sensor (4SR)

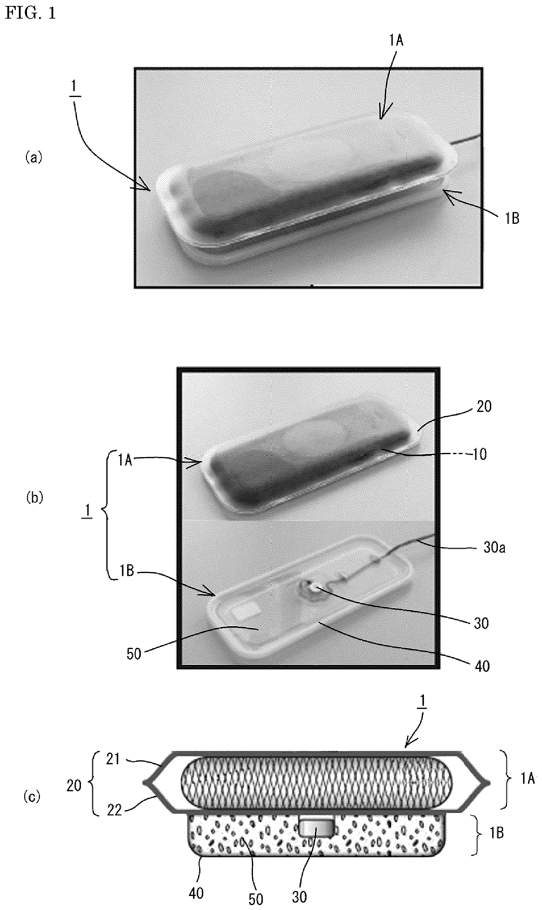

First, based on FIGs. 1(a) to (c), the configuration of a biological signal detection sensor 1 used in this embodiment will be described. The biological signal detection sensor 1 of this embodiment has a stacked structure of an air pack 1A and a gel pack 1B. The air pack 1A includes a three-dimensional knitted fabric (3D net) 10 and a housing film 20 housing the three-dimensional knitted fabric (3D net) 10 in a sealed manner. The gel pack 1B has a microphone 30 fixedly disposed in its case 40 and has a gel 50 filled around the microphone 30.

The three-dimensional knitted fabric 10 is formed of a pair of ground knitted fabrics disposed apart from each other and a connecting yarn connecting the ground knitted fabrics. For example, the ground knitted fabrics each can be formed to have a flat knitted fabric structure (fine meshes) continuous both in a wale direction and a course direction using yarns of twisted fibers or to have a knitted fabric structure having honeycomb (hexagonal) meshes. The connecting yarn imparts certain rigidity to the three-dimensional knitted fabric so that one and the other of the ground knitted fabrics are kept at a predetermined interval. Therefore, applying tension in the planar direction makes it possible to cause string vibration of the yarns of the facing ground knitted fabrics forming the three-dimensional knitted fabric or of the connecting yarn connecting the facing ground knitted fabrics. Accordingly, cardiovascular sound/vibration being a biological signal causes the string vibration and is propagated in the planar direction of the three-dimensional knitted fabric.

As the material of the yarns forming the ground knitted fabrics or the connecting yarn in the three-dimensional knitted fabric, various materials are usable, and examples thereof include synthetic fibers and regenerated fibers such as polypropylene, polyester, polyamide, polyacrylonitrile, and rayon, and natural fibers such as wool, silk, and cotton. These materials each may be used alone or any combination of these may be used. Preferably, the material is a polyester-based fiber represented by polyethylene terephthalate (PET), polybutylene terephthalate (PBT), and the like, a polyamide-based fiber represented by nylon 6, nylon 66, and the like, a polyolefin-based fiber represented by polyethylene, polypropylene, and the like, or a combination of two kinds or more of these fibers. Further, the shapes of the ground yarns and the connecting yarn are not limited either, and any of round cross-section yarns, modified cross-section yarns, hollow yarns, or the like may be used. Carbon yarns, metallic yarns, and so on are also usable.

The following products are usable as the three-dimensional knitted fabric, for instance.
(a) product number: 49013D (manufactured by Sumie Textile Co., Ltd.), 10 mm thickness
   material:
   front-side ground knitted fabric ... twisted yarn of two polyethylene terephthalate fiber false twisted yarns with 450 decitexes/108 f
   rear-side ground knitted fabric ... twisted yarn of two polyethylene terephthalate fiber false twisted yarns with 450 decitexes/108 f
   connecting yarn ................. polytrimethylene terephthalate monofilament with 350 decitexes/1 f
(b) product number: AKE70042 (manufactured by Asahi Kasei Corporation), 7 mm thickness
(c) product number: T28019C8G (manufactured by Asahi Kasei Corporation), 7 mm thickness

The three-dimensional knitted fabric 10 is covered with the housing film 20. In this embodiment, the housing film 20 is composed of two films 21, 22 made of a synthetic resin, they are arranged to cover the front surface and the rear surface of the three-dimensional knitted fabric 10, and peripheral edge portions of the films 21, 22 are bonded by welding or the like. Consequently, the three-dimensional knitted fabric 10 is housed in the housing film 20 in a sealed manner. Note that when the peripheral edge portions of the films 21, 22 are bonded, they are preferably bonded such that the films 21, 22 slightly press the three-dimensional knitted fabric 10 in the thickness direction. This increases the tension of the three-dimensional knitted fabric 10 to easily cause the string vibration of the yarns forming the three-dimensional knitted fabric 10.

The case 40 is attached to the outer side of the housing film 20, and the microphone 30 is disposed in the case 40. The gel 50 as an external disturbance mixture preventing member is filled in the case 40 to surround the microphone 30. The case 40 is made of a synthetic resin and has the function of preventing acoustic vibration propagated to the microphone 30 from spreading out, and the gel 50 inhibits external vibration from being captured by the microphone 30. A cord 30a that carries detected acoustic vibration data as an electric signal is connected to the microphone 30.

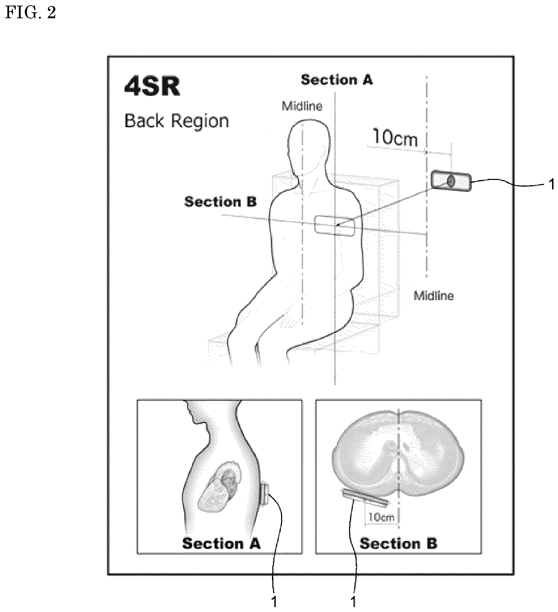

Including the three-dimensional knitted fabric and so on, the biological signal detection sensor 1 is capable of measuring an acoustic pulse wave (APW) amplified by a stochastic resonance phenomenon. When used, the biological signal detection sensor 1 is attached to any of various regions of a measurement target, for example, his/her back, chest, lumber, or the like. Vibration on the body surface is propagated to the housing film 20 and the three-dimensional knitted fabric 10 to be captured by the microphone 30, but when used, the biological signal detection sensor 1 is not limited to be pasted directly on the skin surface but may be attached to the surface of clothing, a seat back of a chair, or the like. Note that in later-described experiment examples, an acoustic pulse wave propagated from the posterior chest surface of a person (R-APW) is collected. Therefore, the biological signal detection sensor 1 is put on clothing, a seat back, or the like such that the microphone 30 is located about 10 cm left of the backbone in the posterior chest surface of the person, as illustrated in FIG. 2,

### - Biological state evaluation device

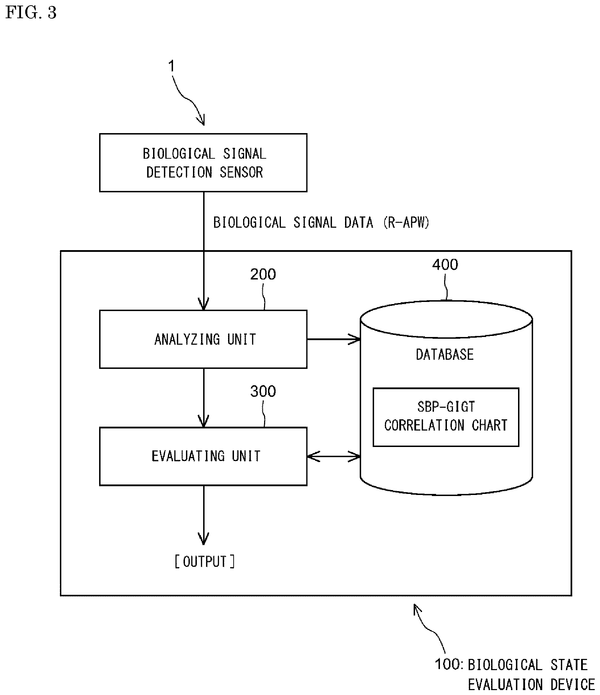

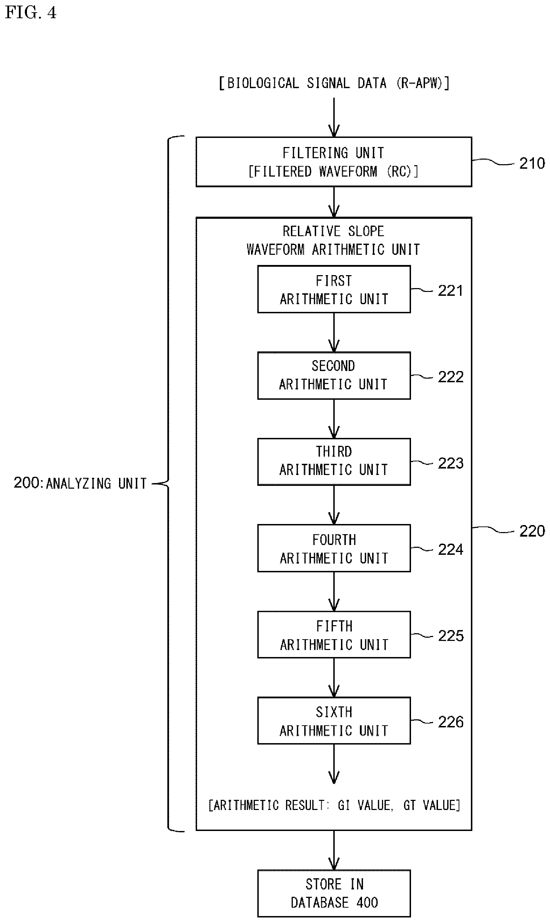

Next, a biological state evaluation device 100 in which a computer program for processing data obtained from the biological signal detection sensor 1 is set will be described based on FIG. 3 and FIG. 4.

The biological state evaluation device 100 processes and analyzes biological signal data obtained by the biological signal detection sensor 1 to evaluate a biological state. The biological state evaluation device 100 is constituted by a computer (including a personal computer, a microcomputer incorporated in a device, and so on) and receives the biological signal data transmitted from the microphone 30 of the biological signal detection sensor 1. It has an analyzing unit 200 and an evaluating unit 300 which perform predetermined processing using the received biological signal data.

In more detail, in the biological state evaluation device 100, a computer program causing the execution of procedures functioning as the analyzing unit 200 and the evaluating unit 300 is stored in a storage unit (including not only a recording medium such as an internal hard disk as the computer (biological state evaluation device 100) but also various removable recording media, a recording medium of another computer connected through communication means, and so on). It further has a database 400 in which the analysis results of the analyzing unit 200 are accumulated and which is referred to at the time of the evaluation by the evaluating unit 300. The database 400 is also stored in a storage unit (including not only a recording medium such as an internal hard disk as the computer (biological state evaluation device 100) but also various removable recording media, a recording medium of another computer connected through communication means, and so on). The biological state evaluation device 100 can be implemented using an electronic circuit having one or more storage circuits in which the computer program functioning as the analyzing unit 200 and the evaluating unit 300 is incorporated.

Further, the computer program can be provided while stored in a recording medium. The recording medium storing the computer program may be a non-transitory recording medium. The non-transitory recording medium is not limited, and examples thereof include recording media such as a flexible disk, a hard disk, CD-ROM, MO (magneto-optical disk), DVD-ROM, and a memory card. Further, the computer program can also be transferred to the computer through a communication line to be installed therein.

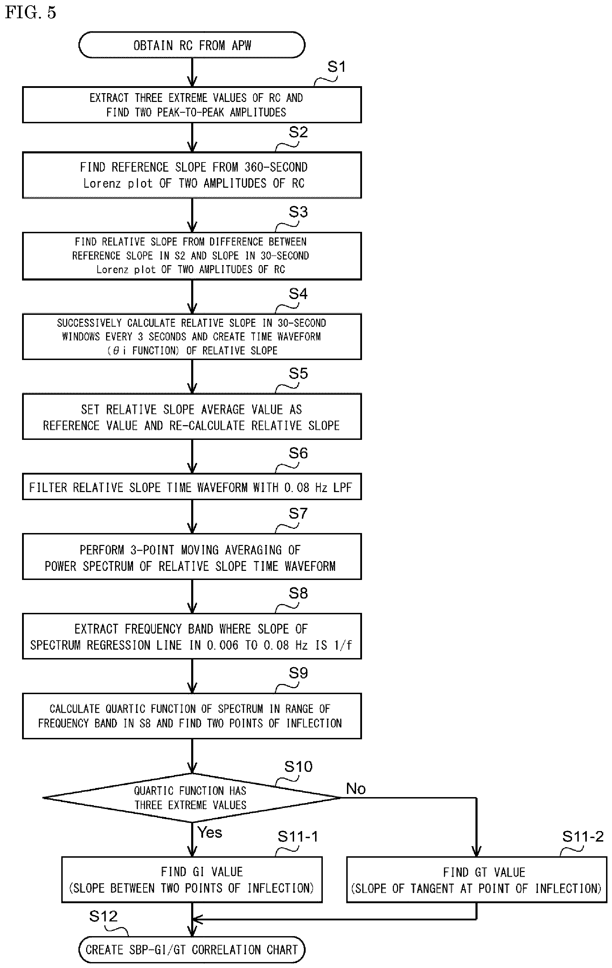

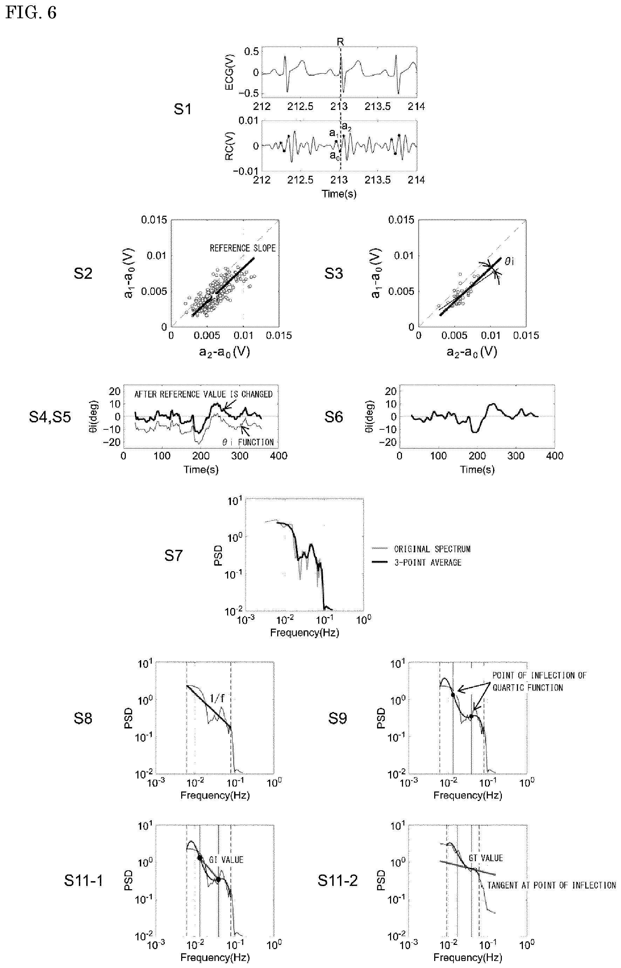

As illustrated in FIG. 4, the analyzing unit 200 has a filtering unit 210 and a relative slope waveform arithmetic unit 220. In more detail, the filtering unit 210 and the relative slope waveform arithmetic unit 220 are configured as programs corresponding to steps S1 to S12 in FIG. 5 and FIG. 6.

The filtering unit 210 is a means for filtering the biological signal data obtained from the biological signal detection sensor 1 (an acoustic pulse wave collected from, preferably, the posterior chest (R-APW) to be amplified by the stochastic resonance phenomenon), with, for example, a bandpass filter whose center frequency is around 20 Hz, preferably, a bandpass filter in a 10 to 30 Hz frequency band. Consequently, a 10 to 30 Hz filtered waveform, that is, a periodic function (Resonance Carrier: RC) having probability distribution whose center frequency is 20 Hz is obtained. A standard heart rate range is around 1 to 1.5 Hz, but in RC, a waveform component with a relatively large total amplitude is captured at an about one-second cycle as illustrated in step S1 in FIG. 6, and thus a cardiac cycle is made apparent.

Further, as illustrated in FIG. 4, the relative slope waveform arithmetic unit 220 has a first arithmetic unit 221, a second arithmetic unit 222, a third arithmetic unit 223, a fourth arithmetic unit 224, a fifth arithmetic unit 225, and a sixth arithmetic unit 226. The first arithmetic unit 221 selects, in RC, three extreme values in a time phase from an atrial systole to a ventricular systole to find two peak-to-peak amplitudes therebetween (S1 in FIG. 5 and FIG. 6). Out of the three extreme values, the middle one is an extreme value immediately before an R-wave time phase in an electrocardiogram and the others are extreme values before and after the middle extreme value, and from these, two amplitudes each between the adjacent extreme values are found. It is possible to obtain the R-wave time phase in the electrocardiogram by measuring electrocardiogram waveform data simultaneously with the measurement by the above-described biological signal detection sensor 1 of the embodiment, but in this case, it is required to put on an electrocardiograph. On the other hand, by capturing atrioventricular valve's closing sound which corresponds to the R-wave time phase in the electrocardiogram, that is, a time phase of a first heart sound, using the biological signal data obtained from the biological signal detection sensor 1, it is possible to identify the R-wave time phase without using an electrocardiograph.

As a means for capturing the time phase of the first heart sound, the means proposed by the present applicant in Japanese Patent Application No. 2020-180964 and Japanese Patent Application No. 2020-180963 is usable. The focal point in this means is to find a boundary frequency (BF) between vibration ascribable to apex beat and vibration ascribable to heart sound from the frequency analysis result of biological signal data, and the use of the boundary frequency makes it possible to extract the vibration ascribable to the heart sound from the biological signal data, and consequently to identify the time phase corresponding to the first heart sound.

The second arithmetic unit 222 is a means using the Lorenz plot method and it executes steps S1 to S6 in FIG. 5 and FIG. 6. An amplitude change of the RC time waveform in the time phase from the atrial systole to the ventricular systole is processed by Lorenz plotting. Specifically, in the plotting, the total amplitudes of the two amplitudes found by the first arithmetic unit 221 are taken on the vertical axis and the horizontal axis. At this time, a slope of a point group plotted over a predetermined measurement time (for example, the total measurement time) is found as a reference slope (S2 in FIG. 5 and FIG. 6). Further, regarding a time shorter than the predetermined measurement time (for example, the total measurement time) (for example, in the case where the total measurement time is 360 seconds, the shorter measurement time is shorter than 360 seconds (for example, thirty seconds)), the same plotting is done using the Lorenz plot method. Next, a slope of a point group plotted in this short window measurement time (short-time point group slope) is found, and a difference between this short-time point group slope and the reference slope is also found as a relative slope (θi) (S3 in FIG. 5 and FIG. 6). The short-time slope is successively found in each time window corresponding to the aforesaid short measurement time with a predetermined overlap ratio. For example, in the case where the overlap ratio is 90%, time windows slid by three seconds in terms of time window second are set, and the short-time slope and the relative slope (θi) are found in each time window. Then, a time waveform of the relative slope (θi) is found (S4 in FIG. 5 and FIG. 6), and a time waveform is further re-calculated using an average value of the relative slopes as a reference value, (S5 in FIG. 5 and FIG. 6).

In the later-described experiment examples (the time period where the reference slope is found is the six-minute total measurement time, and the short-time point group slope is calculated in each 30-second time window with a 90% overlap ratio), the relative slope time waveform is filtered with a 0.08 Hz low pass filter. Consequently, since one point is plotted every three seconds in the case where the time window is thirty seconds and the overlap ratio is 90%, a higher-side frequency is 1/3 Hz, and a Nyquist frequency is 1/6 Hz (0.17 Hz). As for a lower-side frequency, since the total measurement time is six minutes, the window is thirty seconds, and time windows are slid by three seconds, a point group of 110 points is plotted, and therefore, frequency resolution (Δf) is (1/3)/110 = 0.003 Hz. However, on the frequency axis, since the arithmetic mean of three points (0 Hz, 0.003 Hz, 0.006 Hz) is employed, 0.006 Hz is the minimum value. Under the definition that a waveform is identified with five points, the time required for the waveform is twelve seconds. Therefore, 1/12 seconds = 0.08 Hz is a cutoff frequency, and it can be said that a waveform in a 0.08 Hz frequency band or higher is poor in reliability. Therefore, the 0.08 Hz low pass filter is applied to the relative slope time waveform as described above (S6 in FIG. 5 and FIG. 6). The result thus obtained by filtering the relative slope time waveform with the 0.08 Hz low pass filter, that is, the periodic function of the slope variation obtained from the slope of the point group to which the Lorenz plot method is applied (RC Lorenz) is called a θi function in this specification.

The third arithmetic unit 223 performs frequency analysis. Specifically, it is a means for frequency-analyzing the θi function obtained by the second arithmetic unit 222 to represent the result on log-log axes. An analyzed waveform which is the frequency analysis result represented on the log-log axes is smoothed by undergoing moving averaging of totally three points, that is, a given point and points before and after it, and the result is displayed (S7 in FIG. 5 and FIG. 6).

The fourth arithmetic unit 224 draws a regression line between 0.06 and 0.08 Hz for the smoothed analyzed waveform obtained by the third arithmetic unit 223. As this regression line, employed is a regression line whose slope approaches 1/f most while adjustment is made to locate its one end and the other end at positions as close as possible to the lower limit frequency 0.006 Hz and the upper limit frequency 0.08 Hz respectively. Then, the fourth arithmetic unit 224 extracts a frequency band of this employed regression line (S8 in FIG. 5 and FIG. 6).

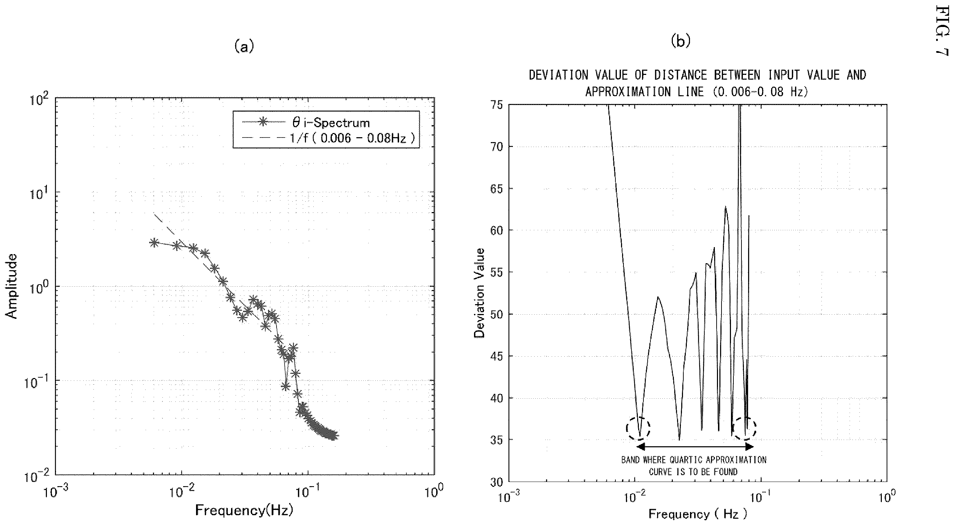

For example, the fourth arithmetic unit 224 draws a regression line between 0.006 and 0.08 Hz for the smoothed analyzed waveform obtained by the third arithmetic unit 223. Specifically, this regression line is first automatically drawn with a 1/f slope as illustrated in FIG. 7(a). Next, as illustrated in FIG. 7(b), a standard deviation of the distances of points of the analyzed waveform from the 1/f regression line is found, a point group of the analyzed waveform in a predetermined frequency band range extracted based on this standard deviation is approximated, and the resultant regression curve represented by a quartic function is found. In this embodiment, a point closest to the regression line (point with the lowest deviation value) on the side of the low frequency 0.06 Hz and a point closest to the regression line (point with the lowest deviation value) on the side of 0.08 Hz are extracted, and a range between the two points is extracted as the frequency band where a graph represented by the quartic function regression curve is to be found.

The fifth arithmetic unit 225 corresponds to step S9 in FIG. 5 and FIG. 6, and it calculates a graph represented by a quartic function regression curve in the frequency band corresponding to one end and the other end of the regression line that the fourth arithmetic unit 224 employed for the smoothed analyzed waveform obtained by the third arithmetic unit 223. The fifth arithmetic unit 225 also finds two points of inflection from the calculated quartic function graph.

The sixth arithmetic unit 226 corresponds to steps S10, S11-1, and S11-2 in FIG. 5 and FIG. 6, and it finds the number of extreme values in the quartic function graph obtained by the fifth arithmetic unit 225 and outputs the final analysis result of the analyzing unit 220. Specifically, the sixth arithmetic unit 226 creates a sign chart and a concavity-convexity chart of the quartic function graph (see FIG. 27 to FIG. 32). In the case where it is determined that the number of extreme values is three ("Yes" in S10 in FIG. 5), it finds a value of a gradient between two points of inflection (Gradient of inflection: GI) of the quartic function graph, and outputs this as the analysis result (S11-1 in FIG. 5 and FIG. 6). In the case where it is determined that the number of extreme values in the quartic function graph is two or less ("No" in S10 in FIG. 5), it finds a value of a gradient of a tangent at a point of inflection (Gradient of tangent: GT) (S11-2 in FIG. 5 and FIG. 6), and outputs this as the analysis result.

Further, in the case where the number of extreme values in the quartic function graph is two or less, it finds a gradient of tangent as follows. Specifically, in the case where the number of the extreme values is two, it decides which one of tangents at points of inflection is to be employed in consideration of whether the point of inflection in a low-frequency band is higher or lower than the maximum value and whether or not both positive and negative gradients of tangents are present. In the case where the number of the extreme values is one and a concave-convex function is present sandwiching a point of inflection, a tangent at a point of inflection that is not the extreme value is employed. The case where the number is two or less will be described in more detail based on a later-described case where the GT value is derived.

The analyzing unit 200 records, in the database 400, the correlation between the value of the gradient between the two points of inflection (GI value) or the value of the gradient of the tangent at the point of inflection (GT value) output as the analysis result and a blood pressure value measured with a blood pressure monitor (brachial) from a subject regarding whom the above values are calculated (S12 in FIG. 5). By recording the correlations regarding many subjects, the correlation data between the blood pressure value measured with the blood pressure monitor and the GI value or the GT value ("SBP-GIGT correlation chart" in FIG. 4) is constructed.

The evaluating unit 300 collates the analysis result in the analyzing unit 200 regarding the biological signal data of an evaluation-target person with the correlation data recorded in the database 400 to evaluate a blood pressure-related biological state of the person.

The determination of the blood pressure-related biological state is, for example, the determination of blood pressure classification. In the determination of the blood pressure classification, the evaluation-target person is classified as at least normotension or hypertension based on the analysis result of the analyzing unit. The blood pressure classification is not limited to this, and the blood pressure can be determined as low or can be determined as optimum or normal. As in the later-described experiment example, in the case where the output analysis result is the GI value, the blood pressure is evaluated as normal, and in the case where it is the GT value, the blood pressure is evaluated as high.

The evaluating unit 300 is capable of not only classifying the blood pressure but also inferring a blood pressure value by collating the GI value or the GT value that is the analysis result, with the correlation data. In this case, as in Patent Document 1 mentioned in Background Art, the biological signal detection sensor 1 of this embodiment is capable of obtaining the biological signal data from a person without restraining the person, and accordingly, it is possible to continuously or intermittently infer the blood pressure value.

Further, the evaluating unit 300 infers whether or not the autonomic nervous system is in disorder, based on whether or not the analysis results of a plurality of biological signal data measured from the same subject at different times present a change conforming to the distribution trend of the correlation data. In particular, using the plurality of biological signal data measured before and after an exercise load, whether or not the autonomic nervous system is in disorder is inferred.

Next, experiments conducted using the biological signal detection sensor 1 and the biological state evaluation device 100 of this embodiment will be described.

### (Experiment Conditions)

### (1) Experiment 1: Blood pressure and R-APW measurement experiment on many unspecified subjects at rest

Participants in this experiment were group A: 77 patients aged 40 to 95 years (73.3±10.3 years) having heart disease, hypertension, diabetes, dyslipidemia, or the like and undergoing treatment at the hospital and group B: subjects in their twenties to thirties (23.1±2.96 years) who were university students. The measurement was conducted for six minutes while the subjects were seated. A reason why the measurement time was set to six minutes is to reduce the influence of rhythm associated with a change in a wakefulness degree or sleepiness during the day and mental workability. The biological signal detection sensor 1 was attached to a seat back of a measurement chair, with its attachment position corresponding to a predetermined position of the posterior chest surface as illustrated in FIG. 2.

In the experiment, in addition to the detection of the acoustic pulse waves by the biological signal detection sensor 1, electrocardiograms (ECGs), finger plethysmograms (PPGs), and phonocardiograms (PCGs) were also measured at the same time. Note that, from the brachial region, a systolic blood pressure (SBP) was measured twice immediately before the start of the measurement and twice after the start of the measurement, and the average value thereof was used. The brachial blood pressure measurement was conducted at a one-minute interval until its variation stably falls within 5 mmHg.

### (2) Experiment 2: Blood pressure and R-APW measurement experiment on a fixed subject at rest

In this experiment, the subject was one male aged 67 years, and during about eleven months from April 2021 to the end of February 2022, the measurement was conducted about 900 times on a five times a week basis. The measurement was conducted while the subject was supine, left lateral recumbent, right lateral recumbent, and seated in a chair, and the measurement time was six to twenty minutes for each posture.

The biological signal detection sensor 1 illustrated in FIG. 1 was used for the measurement, and as illustrated in FIG. 2, acoustic pulse waves measured from the posterior chest surface (R-APW) were collected. As comparative indexes, an electrocardiogram (ECG) and a finger plethysmogram (PPG) were also measured simultaneously with the measurement of R-APW, and a blood pressure was measured before and after the measurement time and when the subject underwent a state change (for example: falling asleep, snoring, or the like) determined through the observation by an experimenter.

### (3) Experiment 3: Blood pressure and R-APW measurement experiment under exercise load (overload)

An exercise stress test was conducted using a treadmill to study the possibility that a GI value or a GT value (whichever of these values is employed, to collectively express them, it is simply expressed as "GIGT value") serves as a parameter of exercise capacity of a cardiocirculatory function. Subjects were six males in their twenties to forties (average age 33.7±7.7 years old). Measured items were an acoustic pulse wave (R-APW), an electrocardiogram, a phonocardiogram, a finger plethysmogram, and a brachial blood pressure (blood pressure measured with a brachial blood pressure monitor). In the experiment, the subjects were kept seated and at rest for twenty minutes in a car seat installed in a room, thereafter ran on the treadmill for about ten minutes, and thereafter were again kept seated and at rest for twenty to sixty minutes. While running on the treadmill, the subjects were given a certain load using Gerkin Fitness Test mounted on the treadmill. Further, before the start of the experiment, before the start of the exercise load, immediately after the cancellation of the exercise load, and after the end of the experiment, a feeling of fatigue was evaluated using Visual Analog Scale (VAS). The blood pressure was measured three times, that is, (1) before the exercise load, (2) immediately after the cancellation of the exercise load, and (3) after twenty to sixty minutes passed after the cancellation of the exercise load. R-APW was measured for six minutes in each interval. The exercise capacity was considered to appear in blood pressure variability, heart rate variability, and R-APW variability in the twenty-minute resting state after the cancellation of the exercise load.

### (Experiment Results)

### (1) Experiment 1: Blood pressure and R-APW measurement experiment on many unspecified subjects at rest

FIG. 8 illustrates correlation data of a systolic blood pressure (SBP) and a GI value or a GT value (SBP-GIGT correlation chart). A regression line for the group of the patients aged 40 to 95 years (73.3±10.3 years) in group A (gray point group) was y = 21.495x + 138.34, and a coefficient of determination (R2) was R² = 0.7599.

A regression line for group B was y = 14.782x + 125.29, and a coefficient of determination (R²) was R² = 0.6184. When the calculation was done regarding totally 103 subjects consisting of group A: the subjects aged 40 to 95 years (73.3±10.3 years) and group B: the subjects in their twenties to thirties (23.1±2.96 years), a regression line was y = 21.25x + 136.38, and a coefficient of determination was R² = 0.7224. From this, it is seen that the GI value and the GT value are effective for inferring the systolic blood pressure (SBP).

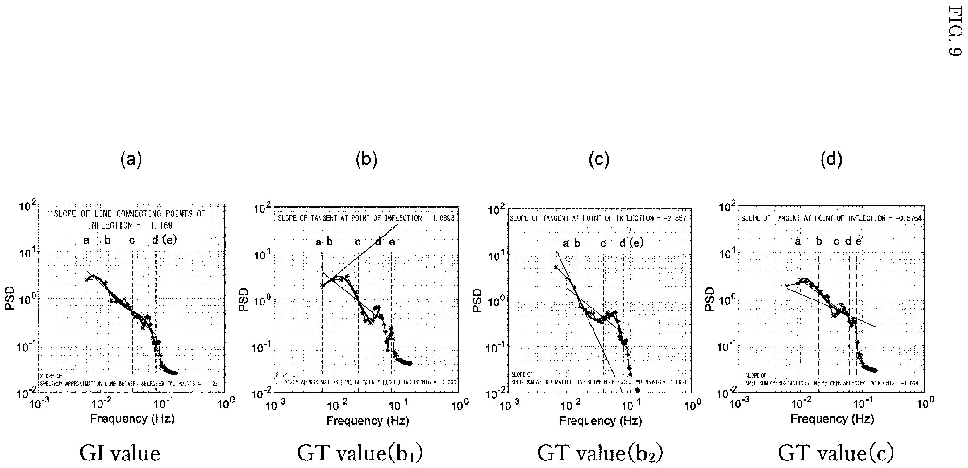

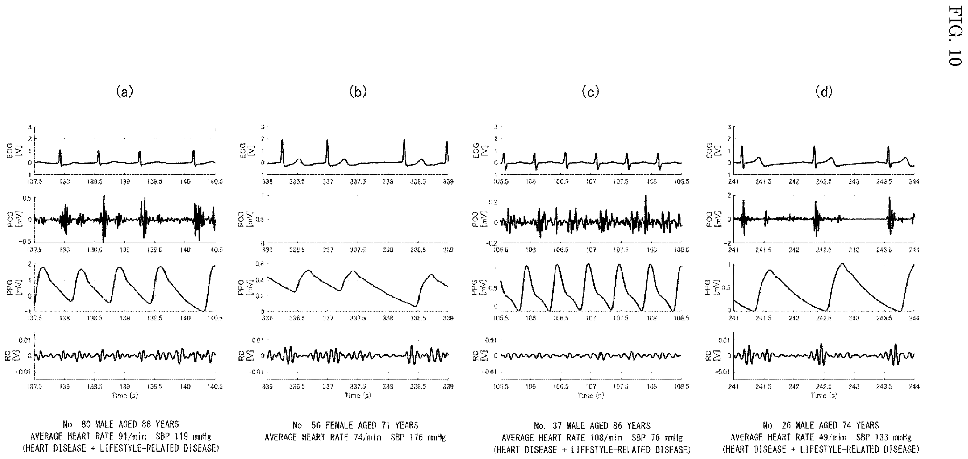

Here, in the analyzing unit 200, the GI value or the GT value is employed depending on the number of the extreme values in the quartic function graph as described above. FIGs. 9 and FIGs. 10 illustrate part of data used in the study of a relation between the number of the extreme values and the GI value or the GT value.

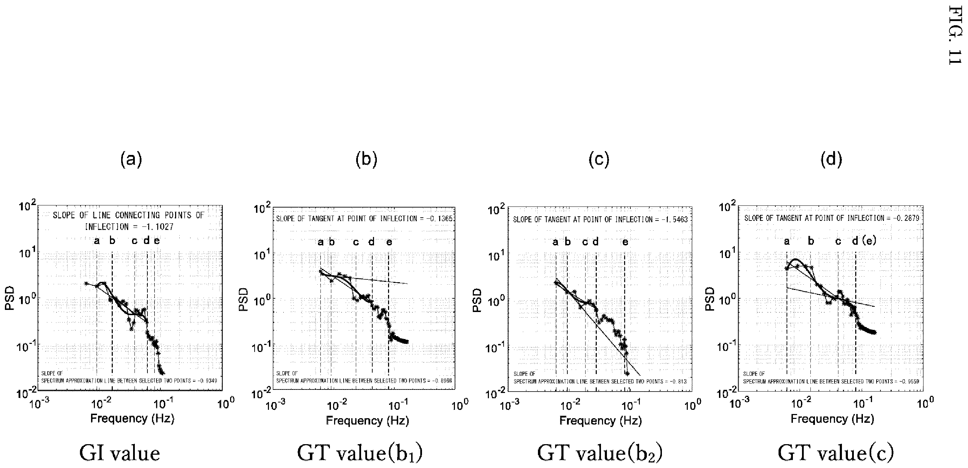

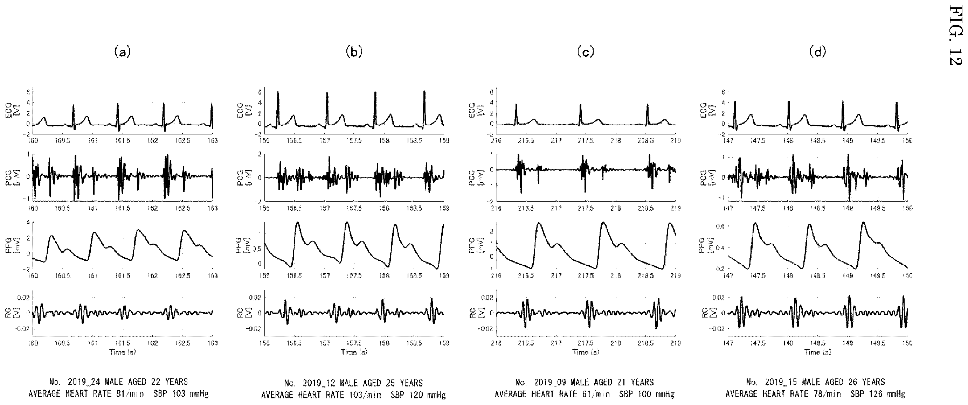

FIGs. 9 are cases where the GI values and the GT values are derived using examples of data of the subjects in group A: the group of the patients having heart disease, hypertension, diabetes, dyslipidemia, or the like. FIGs. 11 are cases where the GI values and the GT values of group B: the group of the subjects in their twenties to thirties are derived. In both cases, time waveforms of ECGs, PCGs, PPGs, and RCs are illustrated in FIGs. 10 which correspond to FIGs. 9 and in FIGs. 12 which correspond to FIGs. 11.

As illustrated in FIG. 9(a) and FIG. 11(a), in the case where the number of extreme values in a quartic function graph is three, a concave-convex function is present sandwiching two points of inflection, and therefore, a gradient connecting the two points of inflection is employed, and a value of the gradient is defined as the GI value.

On the other hand, as for the GT value which is a value of a gradient of a tangent at a point of inflection, there are three types of tangents, that is, b(b1, b2) tangents and a "c" tangent depending on the position of the point of inflection as illustrated in FIGs. 9 and FIGs. 11. The following describes determination criteria.

In the case where there are two extreme values, a point of inflection in a low-frequency band is on a lower side than the maximum value and there are + and - gradients of tangents, the gradient of the tangent on the + side is employed. This is the b1 tangent (FIG. 9(b), FIG. 11(b)).

In the case where a point of inflection in a low-frequency band is on a higher side than the maximum value, a gradient of a tangent at a point of inflection that is not the extreme value is employed. This is the case of the b2 tangent (FIT. 9(c), FIG. 11(c)).

In the case where there is an extreme value and a concave-convex function is present sandwiching a point of inflection, a tangent at a point of inflection that is not the extreme value is employed. This is the c tangent (FIG. 9(d), FIG. 11(d)).

Therefore, depending on the shape of the quartic function graph set for the analyzed waveform, a gradient value of the b1 tangent, the b2 tangent, or the c tangent is employed as the GT value.

Table 1 shows the verification result for normotension (NT) and hypertension (HT) of 130 systolic blood pressure or more using the GI value and the GT value, regarding group A: the group of the patients aged 40 to 95 years (73.3±10.3 years). A p-value was 0.05 or less, indicating that the determination using the GI value and the GT value is effective for determining hypertension, and a negative predictive value (NPV) was high.

**[Table 1]**

| SBP | Gradient | |
|---|---|---|
| | Infection | Tangent |
| ~129 | 56 | 3 |
| 130~ | 0 | 18 |
| p = | 4.28E-14 | |

Table 2 shows the result of determining optimum blood pressure and normotension using the GI value and the GT value, regarding group B: the group of the subjects in their twenties to thirties. In this case as well, a p-value was 0.05 or less, and a negative predictive value was also high. Since y-intercepts are different in the case where group A and group B were considered as different populations in the study as illustrated in FIG. 8, different determination criteria were used. In the case of a young and healthy population like the subject group B, it is also possible to use the GI value and the GT value for the determination of an optimum blood pressure and normotension according to the y-intercept.

**[Table 2]**

| SBP | Gradient | |
|---|---|---|
| | Infection | Tangent |
| <120 | 19 | 2 |
| 120~ | 0 | 5 |
| p = | 4.15E-05 | |

In the case where the GI values and the GT values of the subjects of group B are included in the verification in Table 1 as well, there is no data classified as false negative, and the use of the GI value and the GT value remains suitable for determining hypertension.

As is seen from the foregoing, the evaluating unit 300 is capable of determining a blood pressure category such as normotension or hypertension by collating the GI value or the GT value of an evaluation-target person, which value is the analysis result that the analyzing unit 200 obtains by analyzing the biological signal data of the evaluation-target person, with the SBP-GIGT correlation chart. It is also capable of inferring the systolic blood pressure value from the GI value or the GT value obtained from the analyzing unit 200. In addition, as is apparent from Table 1 and Table 2, the negative predictive value is very high, showing that it is possible to determine a blood pressure category and infer a blood pressure value more accurately than conventionally.

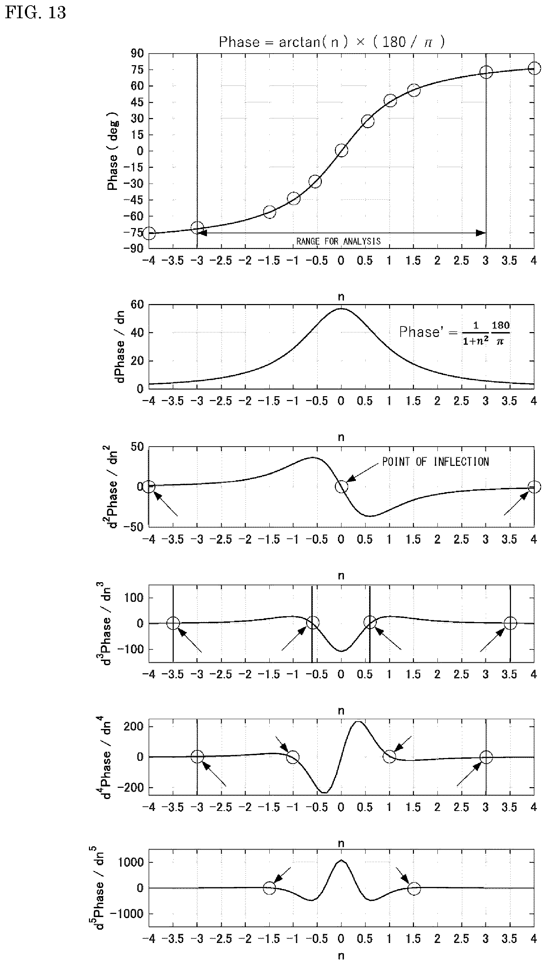

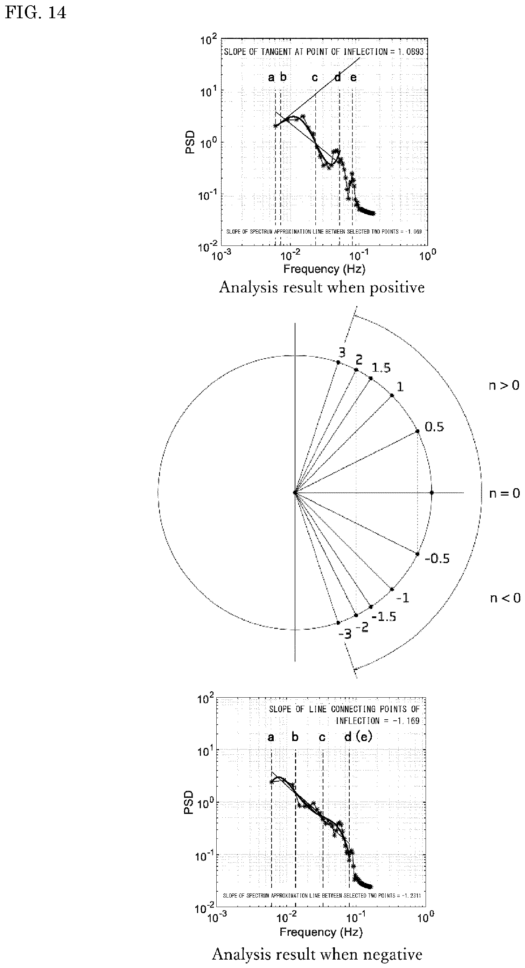

Here, in FIG. 13, an independent variable n on the horizontal axis which is a differential coefficient (a gradient on the log-log axes: a gradient between points of inflection (GI value), a gradient of a tangent at a point of inflection (GT value)) of a quartic function graph and an inverse trigonometric function of n on the vertical axis are represented as Degree (Phase). It is seen that dense data is made sparse so that its trend can be easily seen. In FIG. 14, relations between Phase and the GI and GT values are represented by a fixed point n on the circumference of a polar coordinate system and a rotation angle. Here, it is also seen that data is expanded for easier view. Note that the place indicated by the arrow in the drawing indicates a possibility that a range for analysis falls within ±3 by performing the second, third, fourth, and fifth-order differentiations.

The method of calculating the GI value and the GT value in the flowcharts in FIG. 5 and FIG. 6 is a chaos control method of reducing chaos, and a possibility is indicated that this gave a significant difference to the correlation data of the systolic blood pressure and the GI value or the GT value (SBP-GIGT correlation chart) in FIG. 8.

FIGs. 15 illustrate a correlation chart of a systolic blood pressure and a heart rate (SBP-HR correlation chart) and a correlation chart of the systolic blood pressure and a reciprocal of the heart rate (SBP-1/HR correlation chart) which are created using the data used for creating FIG. 8. Charts where they are on logarithmic scales are also given. In the SBP-HR correlation chart in which the evaluation targets were many unspecified people, no significant correlation was recognized. Even when the logarithmic scales were used, no significant correlation was obtained either.

### (2) Experiment 2: Blood pressure and R-APW measurement experiment on a fixed subject at rest

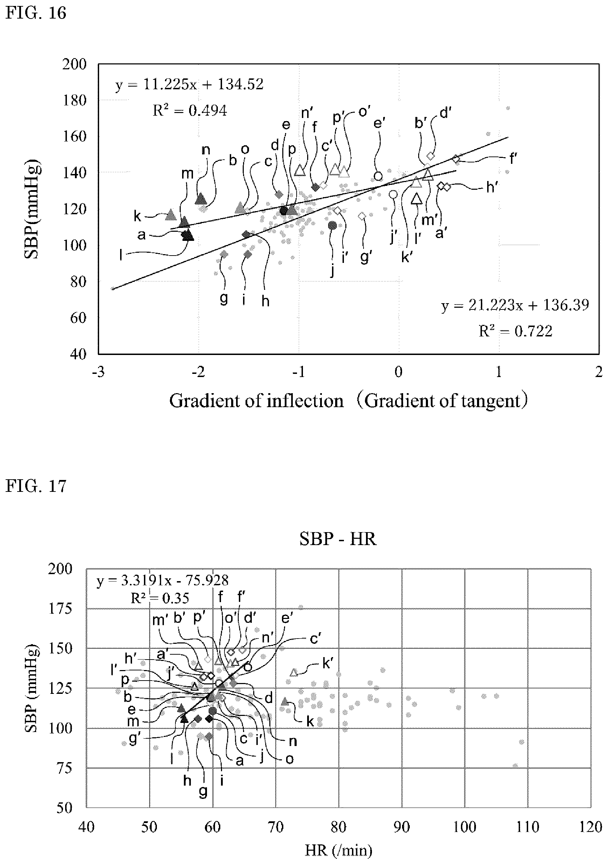

In FIG. 16, out of the measurement results of one subject, data where systolic blood pressure variability of 15 mmHg or more (variability between points indicated by signs a-a', b-b', c-c', d-d', e-e', f-f, g-g', h-h', i-i', and j-j') is recognized during the experiment are superimposed on a data group of many unspecified subjects (gray point group). A slope was that presented in the data group of the subjects in their twenties to thirties in FIG. 8, and a y-intercept substantially approximates that of the subjects aged 20 to 95 years. R² = 0.494, and the correlation data of a systolic blood pressure and a GI value or a GT value (SBP-GIGT correlation chart) presented a significant correlation.

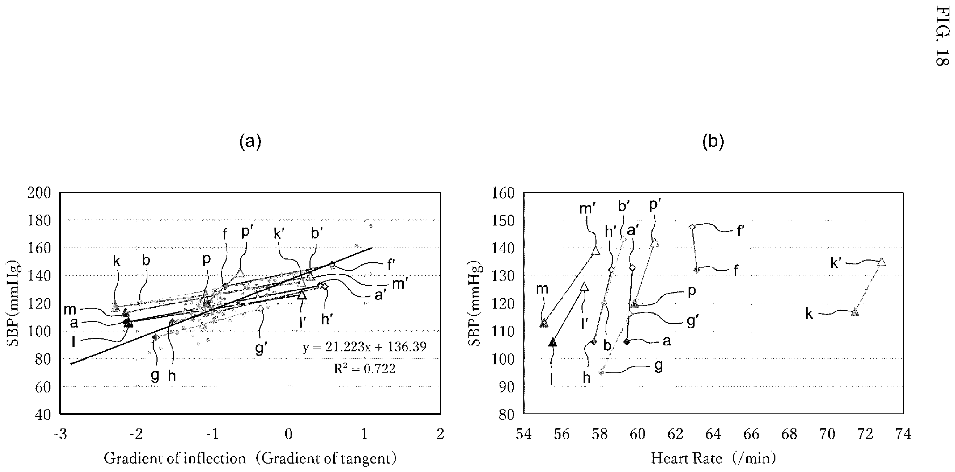

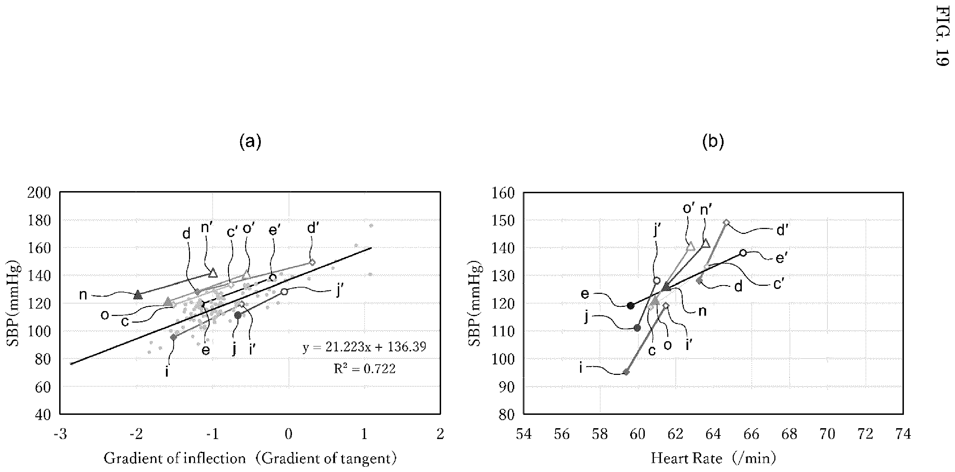

In FIG. 17, on a correlation chart of a systolic blood pressure and a heart rate (SBP-HR correlation chart) of the many unspecified subjects (gray point group), measurement data of the aforesaid subject who underwent blood pressure variability (points indicated by signs a-a', b-b', c-c', d-d', e-e', f-f', g-g', h-h', i-i', j-j', k-k', l-l', m-m', n-n', o-o', and p-p') is superimposed. A regression equation of a person whose blood pressure suddenly changed when the heart rate was 54 to 64/min was y = 3.3191x - 75.928, and a coefficient of determination was R² = 0.35. As for a person whose blood pressure suddenly changed when the heart rate was 70 to 80/min (k-k' data), a slope was also substantially the same value. However, since sample size N is one, this was used as data just for reference. In the same subject at rest, a significant difference was recognized in neither blood pressure variability nor heart rate variability. Therefore, a relation between SBP-GIGT and SBP-HR was studied, and as illustrated in FIGs. 18 and FIGs. 19, the classification was done with a focus on a slope of a regression line of the SBP-GIGT correlation chart.

FIGs. 18(a), (b) illustrate a case group where a slope is small with respect to the regression line of the SBP-GIGT correlation chart when the blood pressure variability occurs (points indicated by signs a-a', b-b', f-f', g-g', h-h', k-k', l-l', m-m', and p-p'), out of the data of the aforesaid subject. In FIG. 18(a), the regression line and a coefficient of determination were calculated in the SBP-GIGT correlation chart, and in FIG. 18(b), a regression line and a coefficient of determination were calculated in the SBP-HR correlation chart.

FIGs. 19(a), (b) illustrate a collection of case groups where an experimental value moves substantially on the regression line of the SBP-GIGT correlation chart (points indicated by signs c-c', d-d', e-e', i-i', j-j', n-n', and o-o') when the blood pressure variability occurs, out of the data of the aforesaid subject.

In the case group in FIGs. 18(a), (b), the heart rate varied only slightly with respect to the blood pressure variability. In the case group in FIGs. 19(a), (b), as a blood pressure increased or decreased, the heart rate also increased or decreased, and thus the blood pressure variability and the heart rate variability were linked. This shows that the SBP-GIGT correlation chart is influenced by the way the blood pressure and HR vary.

The heart rate variability leads to a change in a cardiac output. The change in the cardiac output is caused not only by the change in heart rate but also by a change in stroke volume. The stroke volume is influenced by two factors having opposite effects. One is energy for the contraction of the ventricle, and the other is an arterial pressure that has to be overcome for blood output. Therefore, a possibility is indicated that the GIGT value is a factor influenced by a factor of the change in the energy for the contraction of the ventricle and reflecting the influences of two factors which are (1) a phenomenon that increasing an end-diastolic pressure to expand a cardiac muscle in a diastole strengthens contractile force (Starling's law of the heart) and (2) a phenomenon that sympathetic nerve hyperactivity or an increase in adrenaline concentration in blood increases contractile force (contractility increase) while keeping the muscle length fixed.

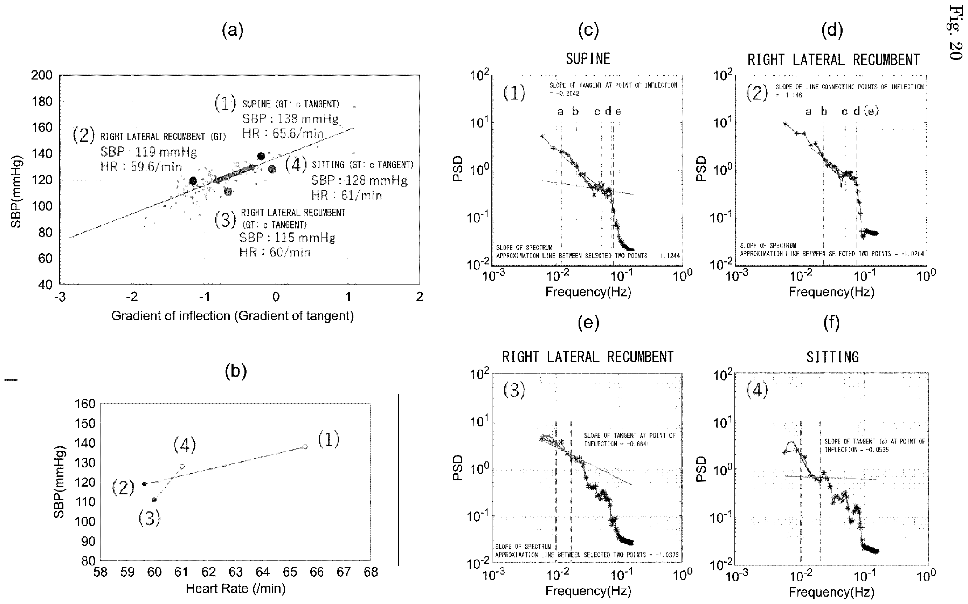

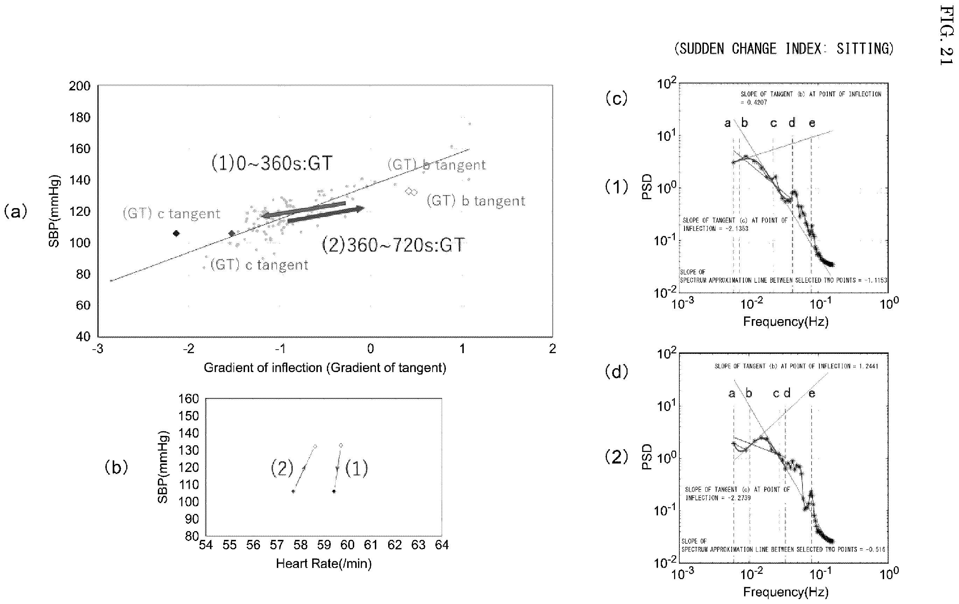

The relation of FIGs. 18 and FIGs. 19 will be explained using FIGs. 20(a) to (f) and FIGs. 21(a) to (d). FIGs. 20(a) to (f) are data of the same subject (subject in his/her sixties) in two cases where his/her systolic blood pressure changed at the time of postural change. FIG. 20(a) is an SBP-GIGT correlation chart, and FIG. 20(b) is an SBP-HR correlation chart. FIG. 20(c) illustrates an analyzed waveform, a quartic function, and a GIGT value found in a supine posture (sign (1) in the drawing) at the time of the postural change from the supine posture to a right lateral recumbent posture, FIG. 20(d) illustrates an analyzed waveform, a quartic function, and a GIGT value found in the right lateral recumbent posture (sign (2) in the drawing) at the time of the postural change from the supine posture to the right lateral recumbent posture, FIG. 20(e) illustrates an analyzed waveform, a quartic function, and a GIGT value found in the right lateral recumbent posture (sign (3) in the drawing) at the time of the postural change from the right lateral recumbent posture to a sitting posture, and FIG. 20(f) illustrates an analyzed waveform, a quartic function, and a GIGT value found in the sitting posture (sign (4) in the drawing) at the time of the postural change from the right lateral recumbent posture to the sitting posture. As is seen from FIG. 20(a), in the correlation data of the systolic blood pressure and the GI value or the GT value (SBP-GIGT correlation chart), experimental values moved on the regression line (from (1) to (2), from (3) to (4)). Then, at the time of the postural change from the supine posture to the right lateral recumbent posture (from (1) to (2)), SBP, HR, and GT value or the GI value decrease in linkage, and at the time of the postural change from the right lateral recumbent posture to the sitting posture (from (3) to (4)), SBP, HR, and the GT value increase in linkage.

FIGs. 21(a) to (d) illustrate a case where the systolic blood pressure suddenly changed during the twelve-minute measurement time during which the same posture (sitting posture) was kept. FIG. 21(a) is an SBP-GIGT correlation chart, and FIG. 21(b) is an SBP-HR correlation chart. FIG. 21(c) illustrates an analyzed waveform, a quartic function, and a GIGT value during the six-minute measurement time from 0 to 360 seconds (sign (1) in the drawing), and FIG. 21(d) illustrates an analyzed waveform, a quartic function, and a GIGT value during the six-minute measurement time from 360 to 720 seconds (sign (2) in the drawing). It is seen from FIG. 21(a) that, when a 15 mmHg sudden change or more occurred during six minutes, experimental values in (1) and (2) both moved so as to cross a regression line of the SBP-GIGT correlation chart. In the SBP-HR correlation chart in FIG. 21(b), HR variability was small with respect to SBP variability both in (1) and (2). Then, it is seen from FIGs. 21(c), (d), in the quartic function used for calculating the GI value or the GT value (GIGT value), two extreme values and two gradients of tangents were confirmed. At this time, the GT value had two gradients, which state is called GT². GT² appeared when SBP suddenly changed. Note that, in both cases of FIGs. 20 and FIGs. 21, experimental values of the GI value and the GT value moved substantially within a range of a scatter plot in accordance with the change in SBP.

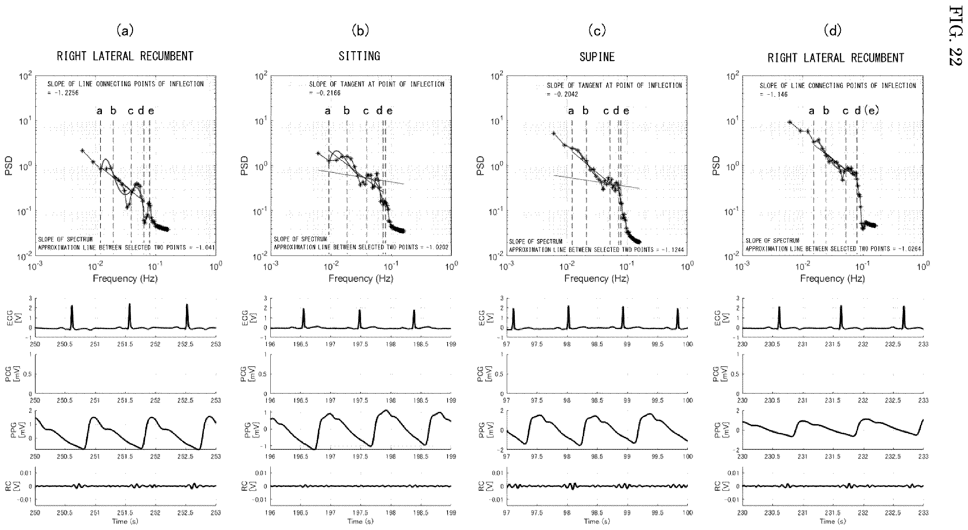

FIGs. 22 and FIGs. 23 illustrate time waveforms of ECGs, PCGs, PPGs, and RCs corresponding to the calculation results of the GIGT value in FIGs. 20 and FIGs. 21. Note that, in FIGs. 23, the calculation results of GIGT values and time waveforms that could be measured under a stable blood pressure in the other postures on the same measurement day are illustrated. When the blood pressure was stable, the GIGT value was the GI value which is the gradient between points of inflection, and when the blood pressure suddenly changed, the GIGT value was the GT value which is the gradient of the tangent at the point of inflection. Therefore, a possibility was indicated that the GT value serves as a signal of a blood pressure increase or a blood pressure sudden change.

Table 3 indicates a possibility that the use of the blood pressure sudden change data in the resting state makes it possible to capture a blood pressure sudden change from GT².

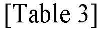

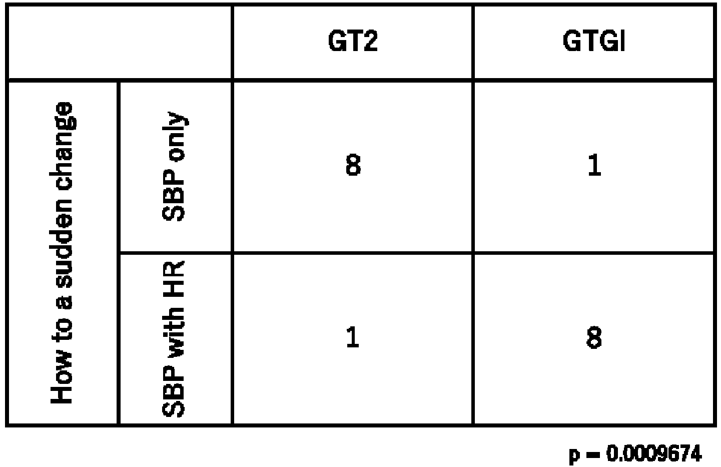

### (3) Experiment 3: Blood pressure and R-APW measurement experiment under exercise load (overload)

FIG. 24 to FIG. 26 are analysis data of a subject in his/her twenties and a subject in his/her forties in the exercise stress test. FIG. 24 illustrates an SBP-GIGT correlation chart, FIGs. 25 illustrate frequency analysis results, and FIG. 26 illustrates an SBP-HR correlation chart. (1) represents the resting state before the exercise load is given, (2) represents a relation between a blood pressure and a heart rate in the resting state immediately after the cancellation of the exercise load, and (3) represents a relation between a blood pressure and a heart rate before and after the resting state during twenty minutes after the cancellation of the exercise load, and they are shown on the SBP-GIGT correlation chart.

In a case having a weak feeling of fatigue in response to the exercise load, SBP increased/decreased as HR increased/decreased. For (1)-1, (1)-2, and (1)-3 in FIG. 24 to FIG. 26, the GIGT values shown in FIG. 27 to FIG. 29 are used. Next, in a case having a strong feeling of fatigue in response to the exercise load, HR increased as SBP increased, but in a decreasing stage, HR did not decrease to a value close to that in the resting state. During a twenty-minute period when the blood pressure suddenly changed, HR decreased from 129/min to 108/min during the initial fourteen minutes, but HR did not decrease during the subsequent six minutes. It is considered that SBP suddenly changed during the twenty-minute period. In FIG. 30 to FIG. 32, if the GT calculation method is used, it is considered that SBP did not greatly decrease but only HR decreased during the initial fourteen minutes, and HR did not decrease and SBP decreased during the subsequent six minutes. These correspond to the trajectories (2)-1, (2)-2, (2)-3, (2)-4 in FIG. 24 and FIG. 26 where the GT value and two GT (GT²) values illustrated in FIG. 30 to FIG. 32 were used. The sudden decrease in PSD in the vicinity of 0.01 Hz indicated a possibility of a decrease in contractility. Due to the way the blood pressure varied and the heart rate varied in the resting state during twenty minutes after the cancellation of the exercise load, the exercise capacity of the subject having a strong feeling of fatigue appeared on the SBP-GIGT correlation chart.

In both the subject having a weak feeling of fatigue in response to the exercise load and the subject having a strong feeling of fatigue in response to the exercise load, the experimental values moved on the SBP-GIGT regression lines. The difference in the degree of the feeling of fatigue appeared in how HR varied as SBP varied. In the case where the variations in the heart rate and SBP were harmonic and after the cancellation of the exercise load, HR returned to 70 to 80/min to which the principle of least energy action is applicable, a possibility was indicated that an increase in cardiac output during the exercise was regulated by the autonomic nerves, and the cardiac output was increased due to an increase in a ventricular end-diastolic volume (EDV) and a decrease in a ventricular end-systolic volume (ESV) ascribable to an increase in an ejection factor.

On the other hand, in the case of the subject having a strong feeling of fatigue in response to the exercise load, HR did not return to 70 to 80/min to which the principle of least energy action is applicable. That is, this is an overload state. In the overload state, a heart rate was close to that in the exercise state, but SBP suddenly changed and further decreased to about 20 mmHg from that in the resting state. A possibility was indicated that even when twenty minutes passed after the exercise load, sympathetic nerve hyperactivity continued, and EDV and ESV became out of balance, and a decrease in stroke volume occurred.

FIG. 27 to FIG. 32 illustrate the calculation results of the GIGT values and time waveforms of ECGs, PCGs, PPGs, and RCs of both subjects illustrated in FIG. 24 to FIG. 26. From the data in FIG. 27 to FIG. 29, in the case of the subject having a weak feeling of fatigue in response to the exercise load, immediately after the cancellation of the exercise load, a large amount of blood is output owing to strong contraction so as to respond to the exercise load, a compensation mechanism worked on a high arterial pressure, a stroke volume increased, and the amplitude of PPG became about twice that in the resting state. Owing to the twenty-minute resting state, HR and SBP both decreased, but the amplitude of PPG remained twice that in the resting state. A reflected wave also increased, which indicates a possibility that the blood pressure decreased due to vasodilation.

On the other hand, in the subject having a strong feeling of fatigue, as is seen from FIG. 30 to FIG. 32, a reflective wave is also superimposed on the amplitude of PPG due to an increase in arterial pressure, and a decrease in the amplitude is small. Even in the state where the arterial pressure decreased when twenty minutes passed from the cancellation of the exercise load, the amplitude of PPG still continued to decrease, which indicates a possibility of a decrease in contractility. This can be inferred also from the fact that the stroke volume decreases even though the arterial pressure decreases and a ventricular pressure easily surpasses the arterial pressure.

It is inferred that, during the exercise load, a lot of energy was consumed to increase the ventricular pressure in an isovolumetric systole, resulting in an increase in the amplitude of RC, but even though the arterial pressure decreased when twenty minutes passed from the cancellation of the exercise load, energy for output accordingly decreased, resulting in the late recovery of the amplitude of PPG.

Here, the above-described experiment results will be studied using a KdV equation as a governing equation.

It is considered that a wave height h used for the solution of the KdV equation is associated with a left ventricular pressure during an atrial systole, a height a is associated with a left ventricular pressure during a ventricular systole, and a differential coefficient of amplitude variation of an RC time waveform made of high-frequency components of an apex beat wave of R-APW (an acoustic pulse wave detected from the posterior chest surface) captures an energy variation associated with an internal pressure variation, a blood flow rate, a blood pressure, and vascular resistance.

The amplitude variation of the RC time waveform having high chaoticity is re-configured to a new time waveform by being given periodicity by the Lorenz plot method, and the GI value and the GT value are created through the frequency analysis thereof. As described above, it has been indicated that the GIGT value and the GT² value serve as parameters indicating the state of the autonomic nervous system control and can be used for the determination of hypertension, and the GT² value can be used for the determination of a blood pressure sudden change. Further, the regression equation derived from the SBP-GIGT correlation chart is usable as the boundary condition of heart rate variability and blood pressure variability. FIG. 33(a) is a feeling of fatigue-GIGT value correlation chart, and FIG. 33(b) is a feeling of fatigue-heart rate correlation chart. The feeling of fatigue-heart rate correlation chart in FIG. 33(b) also indicates a possibility that the feeling of fatigue in response to the exercise load has dependence on the heart rate.

FIG. 34 is an SBP-HR correlation chart where a difference between distribution states in a resting state and a state after the cancellation of the exercise load are shown. The gray point group is data in the resting state, and the point group in darker color than the gray point group and the point group larger in diameter irrespective of the color depth are data after the cancellation of the exercise load. FIG. 35 is an SBP-GIGT correlation chart where the data in the resting state and the exercise stress test are compared. It can be considered that combining FIGs. 33 to FIG. 36 makes it possible to find an overload resting state in the exercise stress test.

Table 4 shows a four-divided map where the overload state is extracted using the exercise stress test data. A possibility was indicated that the overload state can be found from GT².

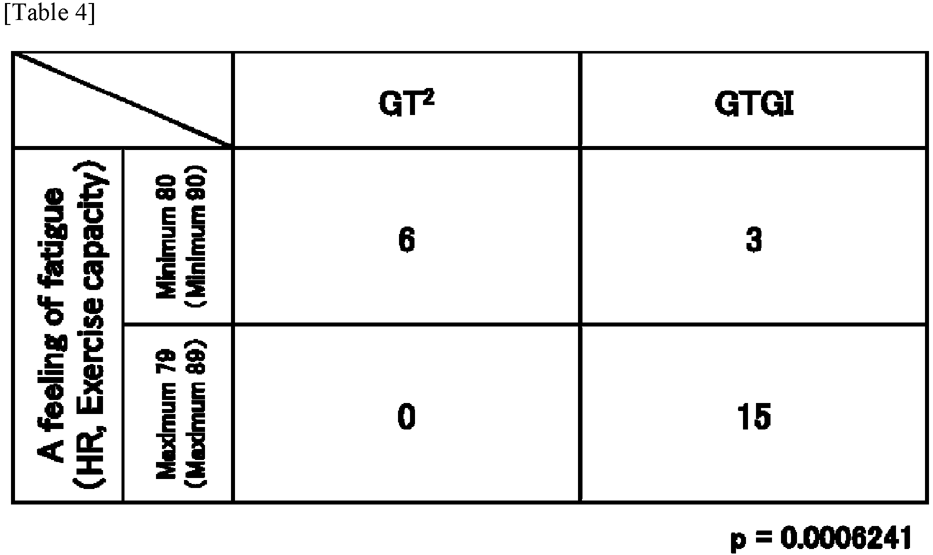

FIG. 36 to FIG. 38 are correlation charts on which temporal changes of SBP and HR in the same person with a strong feeling of fatigue are illustrated as trajectories. It is seen that HR started at 80/min and converged in more than 100/min when twenty minutes (end point) passed after the cancellation of the exercise load. Note that FIRST TIME is a case where SBP at the end point became lower than SBP at the starting point, and the two other cases are cases where SBP was substantially the same at the starting point and the end point. The regression line of the SBP-GIGT correlation chart was substantially the same as the regression line in the resting state. However, from FIGs. 37, it is seen that a decrease in contractility is recognized in (1) and is not recognized in (2) or (3). Therefore, it is indicated that a decrease in blood pressure also causes a decrease in contractility.

FIG. 39 is a regression line of a trajectory of a variation found from an average value in three subjects (subjects in the group with a strong feeling of fatigue), at points including the starting point, the end point, and the intermediate point (immediately after the load cancellation). Averaging resulted in R² = 0.996.

FIG. 40 is a regression line of a trajectory of a variation found from an average value in three subjects (subjects in the group with a weak feeling of fatigue), at three points including the starting point, the end point, and the intermediate point (immediately after the cancellation of the load). The feature of this case lies in that SBP is substantially the same at the starting point and the end point, and averaging resulted in R² = 0.956.

FIG. 41(a) depicts trajectories of the variations in the three persons with a strong feeling of fatigue, and FIG. 41(b) depicts trajectories of the variations in the three persons with a weak feeling of fatigue. A great difference between these lies in heart rate, and in the group with a strong feeling of fatigue, the end point is at 90/min or more, while in the group with a weak feeling of fatigue, the end point is at 90/min or less. This result influences a coefficient of determination of the regression line, resulting in R2 = 0.644 in the group with a weak feeling of fatigue, which is higher in correlation than R2 = 0.3796 in the group with a strong feeling of fatigue.

As is seen from above, the evaluating unit 300 collates the data of the evaluation-target person with the SBP-GIGT correlation chart and is capable of inferring whether or not the autonomic nervous system is in disorder, based on whether or not the data presents a change conforming to the distribution trend of the SBP-GIGT correlation chart. Further, before and after the exercise load, it is also possible to infer whether or not the autonomic nervous system is in disorder as described above.

FIG. 42 is a flowchart illustrating a method of searching for a blood pressure sudden change case by using GT², and FIG. 43 and FIG. 44 are charts illustrating graphs corresponding to steps S21 to S27-2 in FIG. 42. As illustrated in these charts, it is assumed that GT² is first detected (S21). Next, a point where heart rate variability is large is identified based on RRI of an electrocardiogram (S22), and a point where blood pressure variability is large is further detected from blood pressure measurement data (S23). When the heart rate variability and the blood pressure variability are recognized, GT² is calculated (S24). At this time, a point where the blood pressure is at its maximum value is set as GT²⁺, a point where the blood pressure is at its minimum value is set as GT²⁻, and an average value of the blood pressures during six-minute measurement time is set as a GI value.

Next, GT²⁺/GT²⁻/GI values are plotted on an SBP-GIGT correlation chart (S25). Next, intersections of a regression line of the SBP-GIGT correlation chart and the GT²⁺/GT²⁻/GI values are confirmed (S26). In the case of "Yes" in S26, it is confirmed that both the heart rate and the blood pressure value have suddenly changed (S27-1), and a signal indicating the possibility of a physical condition sudden change is output (S28-1). In the case of "No" in S26, it is confirmed that the heart rate and the blood pressure value change in linkage (S27-1), and a signal indicating a possibility of overload is output (S28-2).

FIG. 45 is a case where a subject with a blood pressure sudden change is found from the data of the subjects in group A above. Further, in the case represented by the equation y = 16.146x + 134.26 in FIG. 45, tachyarrhythmia was followed by bradyarrhythmia, and at this time, SBP suddenly changed and a physical condition suddenly changed. FIG. 46 is a case represented by the equation y = 13.336x + 137.16 in FIG. 45. This is a case where, when ST depression appeared on ECG and tachyarrhythmia and bradyarrhythmia alternately occurred, SBP suddenly changed and a physical condition sudden change occurred. FIG. 47 is a case represented by the equation y = 22.582x + 128.47 in FIG. 45. This is a case where ST depression appeared on ECG, and though heart rate variability was small, blood pressure variability occurred, and GT² appeared on a concave function. This is a case of false negative.

As shown in Table 5, it has been found that a subject undergoing a blood pressure sudden change can be detected with high accuracy.

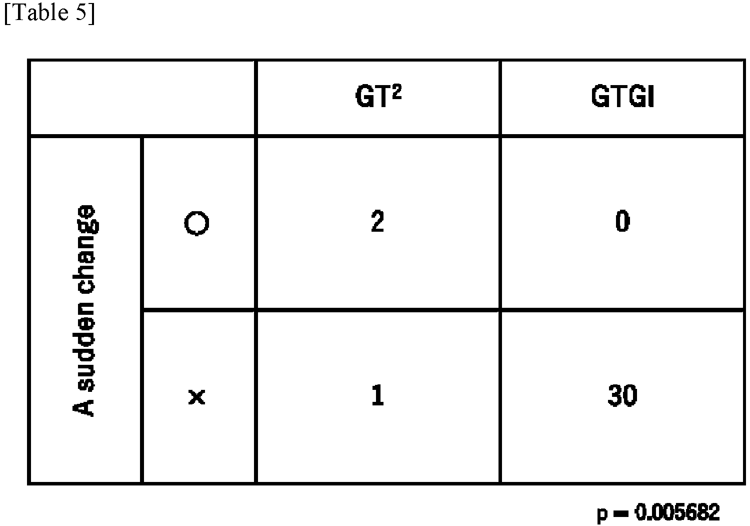

### Explanation of Reference Signs

- 1: biological signal detection sensor
- 10: three-dimensional knitted fabric
- 20: housing film
- 30: microphone
- 40: case
- 50: gel
- 100: biological state evaluation device
- 200: analyzing unit
- 210: filtering unit
- 220: relative slope waveform arithmetic unit
- 300: evaluating unit
- 400: database

## Claims

1. A biological state evaluation device comprising:
an analyzing unit that analyzes biological signal data as sound/vibration information that is propagated from a surface of a body of a person through a three-dimensional knitted fabric of a biological signal detection sensor having the three-dimensional knitted fabric and a microphone, to be detected by the microphone while the biological signal detection sensor is in contact with the body; and
an evaluating unit that accesses a database in which correlation data of analysis results found by the analyzing unit and blood pressure values found with a blood pressure monitor is constructed in advance, and collates an analysis result found by the analyzing unit regarding the biological signal data of an evaluation-target person with the correlation data to evaluate a biological state of the person in relation to a blood pressure,
wherein the analyzing unit analyzes the biological signal data, finds an analyzed waveform regarding heart rate variability information to represent the analyzed waveform on log-log axes, sets a quartic function for the analyzed waveform, finds a gradient between two points of inflection or a gradient of a tangent at a point of inflection depending on the number of extreme values in the quartic function, and outputs one of the gradients as the analysis result.

2. The biological state evaluation device according to claim 1,
wherein, based on whether the analysis result of the biological signal data of the evaluation-target person corresponds to the gradient between the two points of inflection or to the gradient of the tangent at the point of inflection, the evaluating unit determines blood pressure classification including at least distinction between normotension and hypertension.

3. The biological state evaluation device according to claim 1,
wherein the evaluating unit collates a value of the gradient between the two points of inflection or the gradient of the tangent at the point of inflection that is the analysis result of the biological signal data of the evaluation-target person, with the correlation data, and infers a blood pressure value.

4. The biological state evaluation device according to claim 1,
wherein the evaluating unit infers whether or not an autonomic nervous system is in disorder, based on whether or not analysis results of a plurality of biological signal data measured from the evaluation-target person at different times present a change conforming to a distribution trend of the correlation data.

5. The biological state evaluation device according to claim 4,
wherein whether or not the autonomic nervous system is in disorder is inferred, using the plurality of biological signal data measured before and after an exercise load.

6. The biological state evaluation device according to claim 1,
wherein, in a case where the number of the extreme values in the quartic function is three and a concave-convex function is present sandwiching two points of inflection, the analyzing unit employs a gradient between the two points of inflection as the analysis result, and in a case where the number of the extreme values is two or less, the analyzing unit employs a gradient of a tangent at one of points of inflection as the analysis result.

7. The biological state evaluation device according to claim 6,
wherein, in a case where the number of the extreme values is two, the analyzing unit decides which one of tangents at points of inflection is to be employed in consideration of whether the point of inflection in a low-frequency band is higher or lower than a maximum value and whether or not both positive and negative gradients of tangents are present, and
wherein, in a case where the number of the extreme values is one and a concave-convex function is present sandwiching a point of inflection, the analyzing unit employs a tangent at a point of inflection that is not the extreme value.

8. The biological state evaluation device according to claim 1,
wherein, in a case where the analyzing unit finds through the analysis that the number of the extreme values in the quartic function is two and there are two gradients of tangents,
the evaluating unit
evaluates the biological state as having a possibility of an overload state in a case where it is confirmed that heart rates or blood pressures obtained at a predetermined time interval have a predetermined difference or more, and
evaluates the biological state as having a possibility of a physical condition sudden change in a case where it is confirmed that the heart rates and the blood pressures obtained at the predetermined time interval both have the predetermined difference or more.

9. The biological state evaluation device according to claim 1,
wherein the analyzing unit finds, as the heart rate variability information, two peak-to-peak amplitudes from extreme values included in time phases of an atrial systole and a ventricular systole, finds a reference slope from a Lorenz plot in a reference time range by using a Lorenz plot method, successively finds, in predetermined time windows with a predetermined overlap ratio, a relative slope which is a difference between a slope of each Lorenz plot created every predetermined time shorter than the reference time range and the reference slope, applies predetermined-frequency filtering to a time waveform of the obtained relative slope, frequency-analyzes a time waveform resulting from the filtering, converts a result of the frequency analysis to the analyzed waveform represented on the log-log axes, extracts a frequency band where a slope of a regression line of the analyzed waveform is close to 1/f, and finds the quartic function for a point group of the analyzed waveform in a range of the extracted frequency band.

10. A biological state evaluation method of causing a computer to analyze biological signal data as sound/vibration information that is propagated from a surface of a body of a person through a three-dimensional knitted fabric of a biological signal detection sensor having the three-dimensional knitted fabric and a microphone, to be detected by the microphone while the biological signal detection sensor is in contact with the body, to evaluate a biological state of the person, the method comprising:
a procedure for analyzing the biological signal data, finding an analyzed waveform regarding heart rate variability information to represent the analyzed waveform on log-log axes, setting a quartic function for the analyzed waveform, finding a gradient between two points of inflection or a gradient of a tangent at a point of inflection depending on the number of extreme values in the quartic function, and outputting one of the gradients as an analysis result; and
a procedure for referring to correlation data, which is stored in a database in advance, of analysis results found through the execution of the analyzing procedure and blood pressure values found with a blood pressure monitor, and collating an analysis result of the biological signal data of an evaluation-target person with the correlation data to evaluate a biological state of the evaluation-target person in relation to a blood pressure.

11. The biological state evaluation method according to claim 10,
wherein, in the procedure for evaluating the biological state,
based on whether the analysis result of the biological signal data of the evaluation-target person corresponds to the gradient between the two points of inflection or to the gradient of the tangent at the point of inflection, blood pressure classification including at least distinction between normotension and hypertension is determined.

12. The biological state evaluation method according to claim 10,
wherein, in the procedure for evaluating the biological state,
a value of the gradient between the two points of inflection or the gradient of the tangent at the point of inflection that is the analysis result of the biological signal data of the evaluation-target person is collated with the correlation data, and a blood pressure value is inferred.

13. The biological state evaluation method according to claim 12,
wherein, in the procedure for evaluating the biological state,
whether or not an autonomic nervous system is in disorder is inferred based on whether or not analysis results of a plurality of biological signal data measured from the evaluation-target person at different times present a change conforming to a distribution trend of the correlation data.

14. The biological state evaluation method according to claim 13,
wherein whether or not the autonomic nervous system is in disorder is inferred, using the plurality of biological signal data measured before and after an exercise load.

15. The biological state evaluation method according to claim 13,
wherein, in a case where it is found through the analysis that the number of the extreme values in the quartic function is two and there are two gradients of tangents,
in the procedure for evaluating the biological state,
the biological state is evaluated as having a possibility of an overload state in a case where it is confirmed that heart rates or blood pressures obtained at a predetermined time interval have a predetermined difference or more, and
the biological state is evaluated as having a possibility of a physical condition sudden change in a case where it is confirmed that the heart rates and the blood pressures obtained at the predetermined time interval both have the predetermined difference or more.

16. A computer program causing a computer to analyze biological signal data as sound/vibration information that is propagated from a surface of a body of a person through a three-dimensional knitted fabric of a biological signal detection sensor having the three-dimensional knitted fabric and a microphone, to be detected by the microphone while the biological signal detection sensor is in contact with the body, to evaluate a biological state of the person, the program causing the computer to execute:
a procedure for analyzing the biological signal data, finding an analyzed waveform regarding heart rate variability information to represent the analyzed waveform on log-log axes, setting a quartic function for the analyzed waveform, finding a gradient between two points of inflection or a gradient of a tangent at a point of inflection depending on the number of extreme values in the quartic function, and outputting one of the gradients as an analysis result; and
a procedure for referring to correlation data, which is stored in a database in advance, of analysis results found through the execution of the analyzing procedure and blood pressure values found with a blood pressure monitor, and collating an analysis result of the biological signal data of an evaluation-target person with the correlation data to evaluate a biological state of the evaluation-target person in relation to a blood pressure,
thereby causing the computer to function as a biological state evaluation device.

17. The computer program according to claim 16,
wherein, in the procedure for evaluating the biological state,
based on whether the analysis result of the biological signal data of the evaluation-target person corresponds to the gradient between the two points of inflection or to the gradient of the tangent at the point of inflection, blood pressure classification including at least distinction between normotension and hypertension is determined.

18. The computer program according to claim 16,
wherein, in the procedure for evaluating the biological state,
a value of the gradient between the two points of inflection or the gradient of the tangent at the point of inflection that is the analysis result of the biological signal data of the evaluation-target person is collated with the correlation data, and a blood pressure value is inferred.

19. The computer program according to claim 18,
wherein, in the procedure for evaluating the biological state,
whether or not an autonomic nervous system is in disorder is inferred based on whether or not analysis results of a plurality of biological signal data measured from the evaluation-target person at different times present a change conforming to a distribution trend of the correlation data.

20. The computer program according to claim 19,
wherein whether or not the autonomic nervous system is in disorder is inferred, using the plurality of biological signal data measured before and after an exercise load.

21. The computer program according to claim 16,
wherein, in the procedure for outputting the analysis result,
in a case where the number of the extreme values in the quartic function is three and a concave-convex function is present sandwiching two points of inflection, a gradient between the two points of inflection is employed as the analysis result, and in a case where the number of the extreme values is two or less, a gradient of a tangent at one of points of inflection is employed as the analysis result.

22. The computer program according to claim 21,
wherein, in the procedure for outputting the analysis result,
in a case where the number of the extreme values is two, it is decided which one of tangents at points of inflection is to be employed in consideration of whether the point of inflection in a low-frequency band is higher or lower than a maximum value and whether or not both positive and negative gradients of tangents are present, and
in a case where the number of the extreme values is one and a concave-convex function is present sandwiching a point of inflection, a tangent at a point of inflection that is not the extreme value is employed.

23. The computer program according to claim 16,
wherein, in a case where it is found through the analysis that the number of the extreme values in the quartic function is two and there are two gradients of tangents,
in the procedure for evaluating the biological state,
the biological state is evaluated as having a possibility of an overload state in a case where it is confirmed that heart rates or blood pressures obtained at a predetermined time interval have a predetermined difference or more, and
the biological state is evaluated as having a possibility of a physical condition sudden change in a case where it is confirmed that the heart rates and the blood pressures obtained at the predetermined time interval both have the predetermined difference or more.

24. The computer program according to claim 16,
wherein, in the procedure for outputting the analysis result,
as the heart rate variability information, two peak-to-peak amplitudes are found from extreme values included in time phases of an atrial systole and a ventricular systole, a reference slope is found from a Lorenz plot in a reference time range by using a Lorenz plot method, a relative slope which is a difference between a slope of each Lorenz plot created every predetermined time shorter than the reference time range and the reference slope is successively found in predetermined time windows with a predetermined overlap ratio, predetermined-frequency filtering is applied to a time waveform of the obtained relative slope, a time waveform resulting from the filtering is frequency-analyzed, a result of the frequency analysis is converted to the analyzed waveform represented on the log-log axes, a frequency band where a slope of a regression line of the analyzed waveform is close to 1/f is extracted, and the quartic function is found for a point group of the analyzed waveform in a range of the extracted frequency band.

25. A computer-readable recording medium in which the computer program according to any one of claims 16 to 24 is recorded.
